# EUROPEAN PATENT SPECIFICATION

(11) **EP 4 654 889 B1**
(45) Date of publication and mention of the grant of the patent: **25.11.2026**
(21) Application number: 24703071.1
(22) Date of filing: 24.01.2024
(51) Int. Cl.: A61B 5/11, G01L 5/22, G06F 3/01

(54) **MECHANICAL STIMULI DETECTION GLOVE, METHOD OF PREPARATION THEREOF, AND MECHANICAL STIMULI DETECTION SYSTEM AND USE THEREOF**
HANDSCHUH ZUR ERKENNUNG MECHANISCHER STIMULI, HERSTELLUNGSVERFAHREN DAFÜR UND SYSTEM ZUR ERKENNUNG MECHANISCHER STIMULI UND VERWENDUNG DAVON
GANT DE DÉTECTION DE STIMULI MÉCANIQUES, SON PROCÉDÉ DE PRÉPARATION, ET SYSTÈME DE DÉTECTION DE STIMULI MÉCANIQUES ET SON UTILISATION

(30) Priority: 26.01.2023 PT 2023118486
(43) Date of publication of application: 03.12.2025
(73) Proprietor: Associação Almascience - Investigação e Desenvolvimento em Celulose para Aplicacoes inteligentes e sustentaveis, 2829-516 Caparica (PT)
(72) Inventor: AVELÃS RESENDE, João, 2825-394 Costa Da Caparica (PT); SOFIA SANTANA DOS SANTOS, Andreia, 2840-547 Aldeia De Paio Pires (PT); FILIPA PEREIRA GASPAR, Diana, 2420-355 Santa Eufémia - Leiria (PT); SOFIA BRIGIDA MATIAS, Mariana, 6200-006 Covilhã (PT); MIGUEL SANTOS SILVA FREIRE, Tomás, 2675-549 Arroja (PT); MIGUEL RIBEIRO VEIGAS, Bruno, 2600-441 Alhandra (PT); SOFIA DIAS LOPES, Alexandra, 2620-047 Olival Basto (PT); MIGUEL CAVACO SANTOS, Wilson, 2675-238 Odivelas (PT)
(74) Representative: Monteiro Alves, Inês
(86) International application number: PCT/IB2024/050665
(87) International publication number: WO 2024/157185

(56) References cited:
- WO-A1-2023/180959
- US-A1- 2017 059 434
- US-A1- 2020 319 039
- US-A1- 2021 252 278
- US-A1- 2022 291 745
- US-A1- 2023 020 138
- US-B2- 11 331 472

## Description

### [Technical Field

The invention pertains to a mechanical stimuli detection glove and a computational system comprising said glove, which may be used in industry, for medical procedures, or any other training or monitoring system in which at least one physical stimulus selected from a group consisting of a pressure, a touch, a strain, and an elongation exerted by the fingers should be monitored.

### Background Art

The Chinese patent application CN110313663A reveals smart gloves, which comprise glove bodies which are provided with capacitive elastic strain sensors, data processing modules, data sending modules and power supplies. The capacitances of the capacitive elastic strain sensors change under the action of stress. The capacitive sensors are fabricated on the surface of the glove substrate, based on an elastic insulating material, and include a first conductive layer, a second conductive layer, an elastic dielectric layer and an elastic encapsulation layer. The material of the elastic dielectric layer includes an elastic polymer material or the like. Further preferably, the elastic insulating layer is made of an elastic material having good adhesion to a textile material, such as thermoplastic elastomer (TPE), thermoplastic polyurethane elastomer rubber (TPU), dimethyl siloxane (PDMS). Other options of elastic insulating material, include one or more of an aliphatic aromatic random copolyester (Ecoflex), a polymerized resin, a silica gel, a rubber, a hydrogel, a polyurethane, and a polyethylene octene coelastomer (POE).

The South-Korean patent application KR20180117890A describes a wearable pressure sensing glove comprising a plurality of pressure sensor parts disposed on the palm side, and a plurality of feedback parts disposed adjacent to each of the plurality of pressure sensor parts. The plurality of pressure sensor portions may include a first electrode layer including conductive fibers, a second electrode layer, and an elastic layer. The first electrode layer and the second electrode layer may be formed of a fabric and the fabric may comprise conductive fibers (either a metal wire or a plain fiber coated with a metal film on the surface). The conductive fibers may be ordinary fibers in which metal particles are dispersed. On the other hand, the elastic layer may include an elastic body and a conductive complex dispersed in the elastic body. Here, the elastic body may be a synthetic fiber or a natural fiber including one selected from the group consisting of polyurethane, nylon, polyethylene terephthalate and polyester, a fiber substrate having a random fiber arrangement such as a foamed foam, a nonwoven fabric, rubber, urethane, and the like.

The North American patent US2014215684A1 describes a pressure sensing glove in which at least five pressure sensors are intrinsic to the glove manufacture, usually sandwiched between layers of the glove, positioned at the palm tip of each finger, with one pressure sensor per finger. Between five to nine pressure sensors are positioned throughout the glove, and the pressure sensors themselves may be capacitive sensors, piezoelectric sensors, piezoresistive sensors, air filled bladder pressure sensors in communication with hollow tubes further connected to electronic pressure sensors, or any other sensor known in the art. The piezoresistive sensors consist of a micro-machined silicon diaphragm with piezoresistive strain gauges diffused into it. An example of a pressure sensor based on a Tekscan "FlexiForce" sensor is given, where this sensor has a polyester film (or polyimide) substrate.

The Chinese patent application CN106445168A describes a pair of intelligent gloves, which comprises a glove base body, a sensor module, a communication module, a processing module, and a power supply module. The sensor assembly at least includes a fingertip pressure sensor (disposed on the corresponding finger pad of the glove base and the palm muscle), a finger curvature sensor (disposed on the finger back of each joint corresponding to the glove base), a spatial distance sensor (mounted on the palm of the glove base and the palm near the edge of the little finger, or the part that needs coordinate positioning and distance measurement), a fingertip track tracking sensor, a palm trail tracking sensor, a muscle movement sensor (mounted on the palm thumb extensor and palpebral muscle corresponding to the glove base) and a fingerprint identification sensor.

The Chinese patent application CN104544640A describes an intelligent glove having a glove body that comprises a finger part, a palm part and a wrist part. The finger part includes a pressure sensor disposed at the tip of each finger part, bending sensors are disposed at the three finger joints of each of the index finger part, the middle finger part, the ring finger part and the little finger part, and bending sensors are disposed on the two finger joints of the thumb part. A bending sensor and a temperature sensor are disposed on the front side of the palm part. A display, a power module and an information processing module are further disposed on the glove body. The pressure sensors, the bending sensors, the temperature sensor, the display and the power module are electrically connected with the information processing module. The pressure sensor, the bending sensor, and the temperature sensor are each a graphene sensor.

The North American patent US20200319039A1 describes a supercapacitive iontronic nanofabric pressure sensor, which uses ionic materials (e.g., ionic gels) in a polymer network, forming a multi-layered, elastic sensor structure. The ionic material is coated on or forms part of the fibers in the fabric, enabling high elasticity and sensitivity. The sensor operates by measuring changes in capacitance at the interface between the ionic material and conductive fibers as pressure is applied, leveraging the formation of an electric double layer for high sensitivity.

### Technical Problem

A drawback of the glove disclosed in the patent application CN110313663A is related to the dielectric physical properties of the used strain or pressure sensor. It is clear to a person skilled in the art that the elastic strain sensors are made of an elastic insulating layer, resulting in an exclusively capacitive sensor. Another disadvantage of the glove revealed in patent application CN110313663A refers to the limited functionality of the textile material, which acts merely as a support material for the sensors.

According to Jia-wen Zhang *et al,* the capacitive sensors are able to detect lower intensity mechanical stimuli, but their performance regarding their sensibility, which is related to the signal gradient per pressure or force unit, is lower than the sensibility of the resistive sensors. On the other hand, the resistive sensors provide an increased resolution for several intensity of mechanical stimuli but are not proper in uses that require a higher limit of detection.

The subject-matter disclosed in the South-Korean patent application KR20180117890A refers to a resistive sensor, wherein the elastic layer of said sensor has a resistance of at least 1 kiloOhm. The sensor disclosed in this prior art document is not suitable for capacitive measurements, which demand higher resistances, in an order of magnitude of MegaOhms. Therefore, achieving these significant higher values of resistive would demand a drastic reduction of the concentration of dispersing metal particles in the conductive fibers, resulting in so low concentrations, that would prevent the flow of an electrical current through said conductive fibers.

The disadvantages of the gloves disclosed in patent documents US2014215684A1, CN106445168A, and CN104544640A are referred to the limited flexibility of the employed substrates, resulting in impairing the native feeling of the user when wearing the pressure sensing glove.

There is a need to develop mechanical stimuli detection gloves comprising mechanical stimuli sensors, able to operate on capacitive and resistive modes, that are flexible and contribute to mimic a native feeling to the user, without requiring the incorporation of hard or stiff and detectable sensing elements. The sensors that are able to operate on both capacitive, and resistive modes will have the optimized performances of each kind of sensor. Combining capacitive and resistive properties in the sensor allows it to detect lower intensity mechanical stimuli, and an increased resolution for several intensity of mechanical stimuli.

### Solution to Problem

The present invention solves the problems of prior art by impregnating a hydrogel in a fabric substrate layer, forming a layer zone impregnated with a hydrogel, which is contacted to electrodes to allow properly the electrical measurement of mechanical stimuli applied over said fabric substrate layer. This fabric layer impregnated with a hydrogel provides a flexible mechanical stimuli sensor. Due to the composition of the hydrogel, it is possible to perform resistance measurements, capacitance measurements, or a combination of both, which allows both the detection of subtle mechanical stimuli and a high resolution of mechanical stimuli with different intensities, which is not easily achieved with only one type of measurement.

The present invention also solves the problems of inflexibility of the gloves known in prior art by connecting the electrodes to conductive lines that are sewn or printed on the glove material, which avoids the use of less flexible connectors between the glove and a computing device.

### Advantageous Effects of Invention

In the present invention, the hydrogels are an essential element of the flexible mechanical stimuli sensor, namely when hydrogels are bio-based polymers or from natural-derived materials, which are viable sensing layers to produce sensing devices given their abundance, low cost, sustainability, recyclability, and flexibility. Moreover, hydrogels are interesting materials for sensing layers as they are flexible, their performance regarding mechanical and sensing properties are easily tuned, so they can be stretchable and biocompatible, and may show self-healing properties.

The mechanical stimuli sensor based on hydrogels are embedded in the glove through a simple connecting process, for instance a sewing process with a regular sewing line. This is possible due to the substrates of the mechanical stimuli sensor being based on fabric, thus compatible with sewing or connection to the material of glove by using an adhesive. By connecting the pressure sensors to a regular non-sensitive glove, and given the material of the sensors' substrate, the flexibility of the original glove is maintained, and the glove is endowed with the ability to detect mechanical stimuli. The flexibility of the sensing elements also allows the user to grasp objects with irregular surfaces without a compromise of the mechanical sensing, since the sensing elements, together with the glove, can shape up to the object being grasped. Furthermore, the user will have a native feeling when wearing the glove, given that the sensing elements exploit similar materials to those used in the glove, for instance fabrics. Therefore, the user will not feel the presence of a stiff sensing element, thus being able to comfortably wear the glove and make natural movements. The sensitive glove becomes able to distinguish and discriminate distinct activities, namely the grasping of objects with grasping force quantification, hand movements, for example hand opening and closing, hand clenching and fisting, or fingers pointing. Moreover, the mechanical sensors based on the hydrogel can also use the original fabric of the glove as a part of the mechanical sensing device, for example a pressure sensing device, both for printing of the conductive lines or for the incorporation of the hydrogel inside the glove fabric.

The mechanical stimuli sensors can operate on both capacitive, and resistive modes, thus having the optimized performances of each kind of operation mode. Combining capacitive and resistive properties in the sensor allows it to detect lower intensity mechanical stimuli, and an increased resolution for several intensity of mechanical stimuli, without the need to have separate resistive and capacitive sensing elements, thus reducing the complexity of the system and the bulkiness of the glove.

The mechanical stimuli detection glove and system are useful in several purposes, for instance, hand and finger pose estimation and motion tracking and force feedback via electrostimulation. This later includes a first application, namely kinesthetic or force feedback, which provides the impression of movement and resistance through the muscles, like the feeling of weight, inertia, or resistance. A second referred application is the tactile feedback, which provides input to the user skin to recreate different sensations, such as shape, texture, thermal, or smoothness. Additionally, the use of a stacked electrode configuration allows for the feedback to be felt at the point of pressure measurement, enabling a natural and intuitive interaction between the user and the mechanical stimuli detection glove according to the invention.

### Brief Description of Drawings

With the purpose of promoting an understanding of the principles in accordance with the embodiments of the present invention, reference will be made to the embodiments illustrated in the figures and to the language used to describe the same. Anyway, it must be understood that there is no intention of limiting the scope of the present invention to the contents of the figures. Any alterations or later changes of the inventive features illustrated herein, and any additional application of the principles and embodiments of the invention shown, which would occur normally for one skilled in the art when reading this description, are considered as being within the scope of the claimed invention.
**Fig.1**

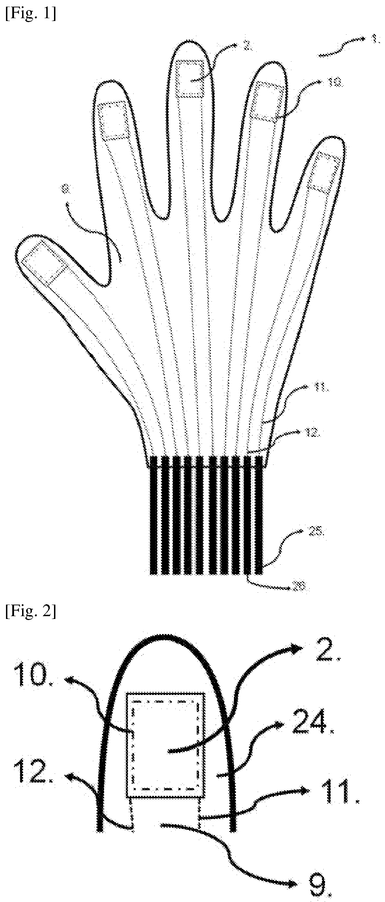

   [Fig.1] illustrates a top view of the mechanical stimuli detection glove according to the invention;
**Fig.2**
   [Fig.2] illustrates a close view of one finger of the mechanical stimuli detection glove connected to a mechanical stimulus sensor;
**Fig.3**

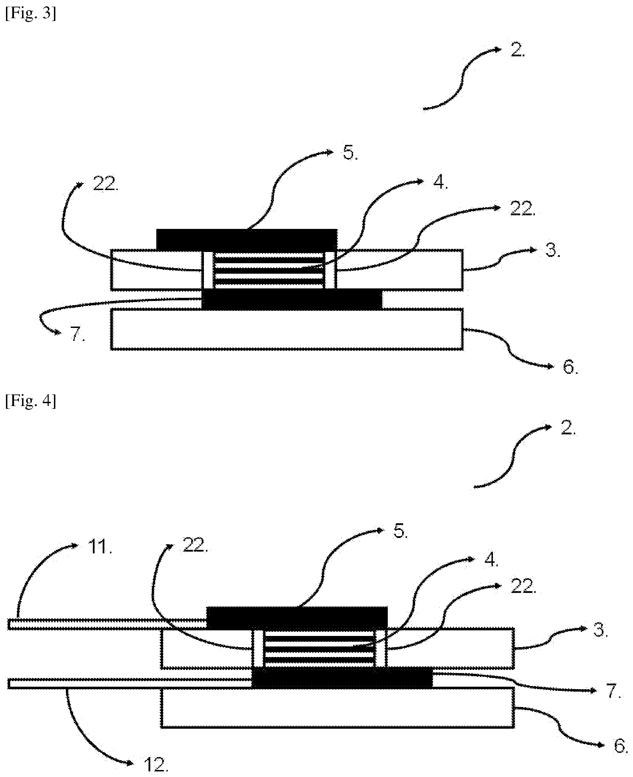

   [Fig.3] illustrates a side view of the mechanical stimuli sensor;
**Fig.4**
   [Fig.4] illustrates a side view of the mechanical stimuli sensor;
**Fig.5**

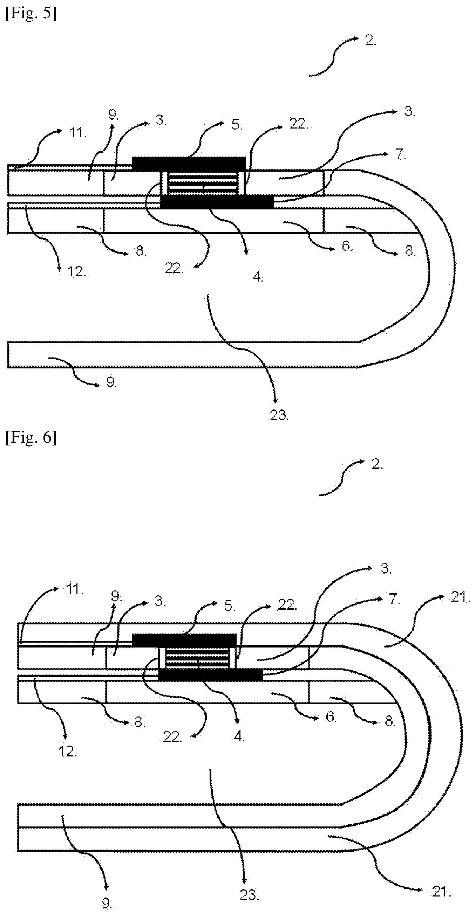

   [Fig.5] illustrates a side view of the mechanical stimuli sensor connected to a glove layer according to a first embodiment of the invention;
**Fig.6**
   [Fig.6] illustrates a side view of the mechanical stimuli sensor connected to a glove layer according to a second embodiment of the invention;
**Fig.7**

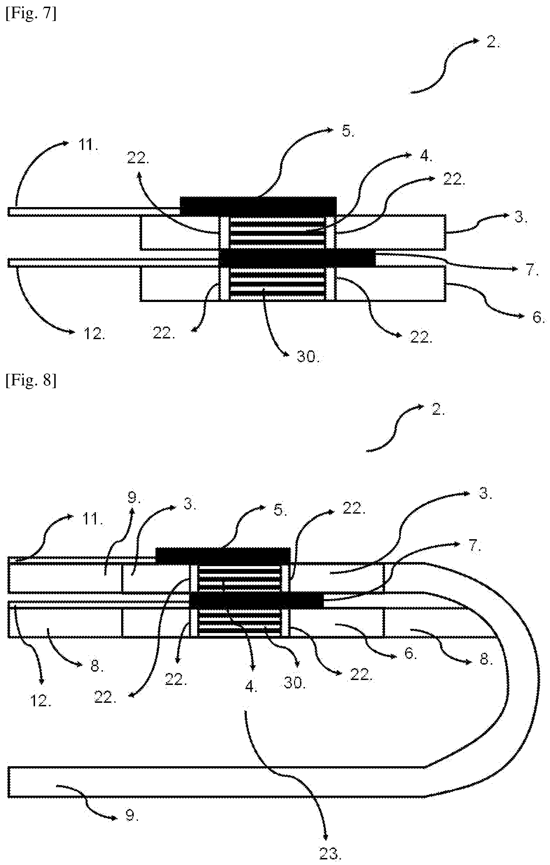

   [Fig.7] illustrates a side view of the mechanical stimuli sensor integrated with a feedback element;
**Fig.8**
   [Fig.8] illustrates a side view of the mechanical stimuli sensor integrated with a feedback element connected to a glove layer according to a third embodiment of the invention;
**Fig.9**

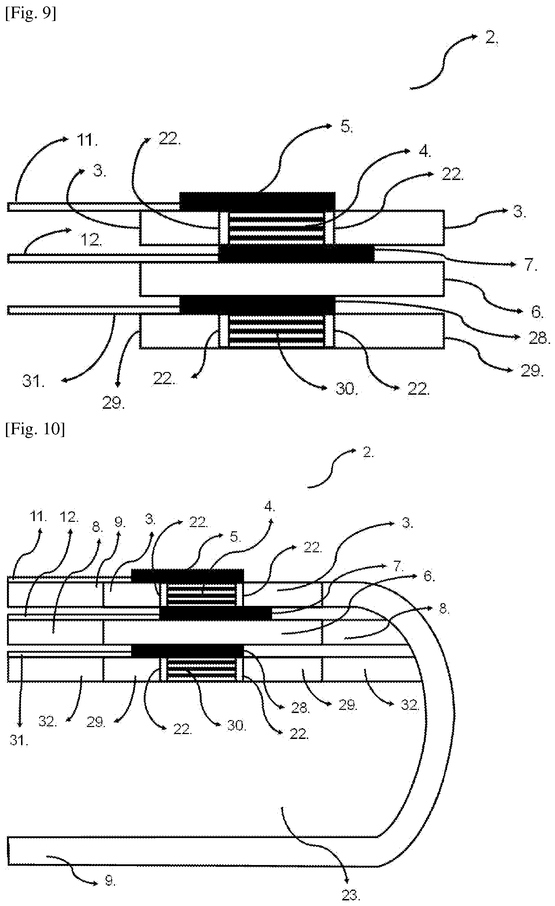

   [Fig.9] illustrates a side view of another embodiment of the mechanical stimuli sensor integrated with a feedback element;
**Fig.10**
   [Fig.10] illustrates a side view of the mechanical stimuli sensor integrated with a feedback element connected to a glove layer according to a fourth embodiment of the invention;
**Fig.11**

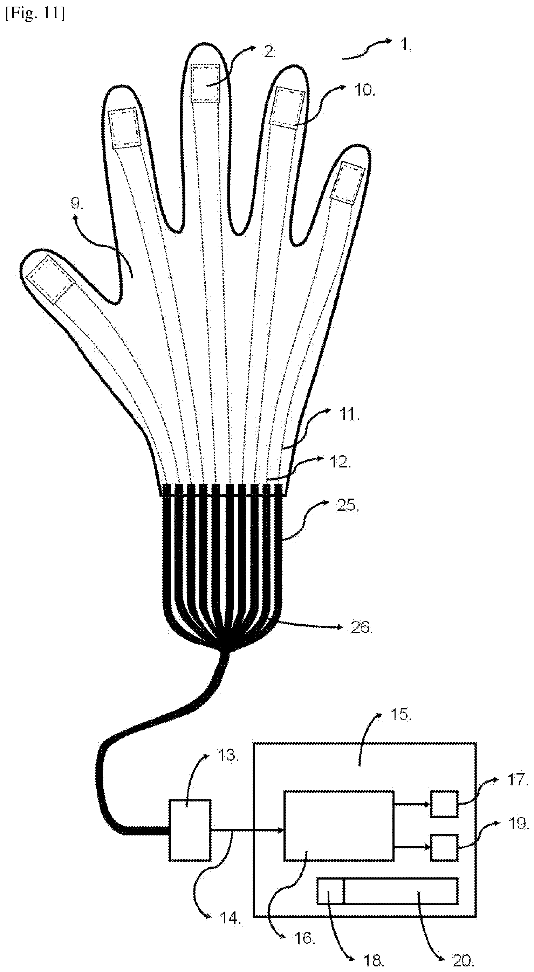

   [Fig.11] illustrates a mechanical stimuli detection system comprising the mechanical stimuli detection glove;
**Fig.12**

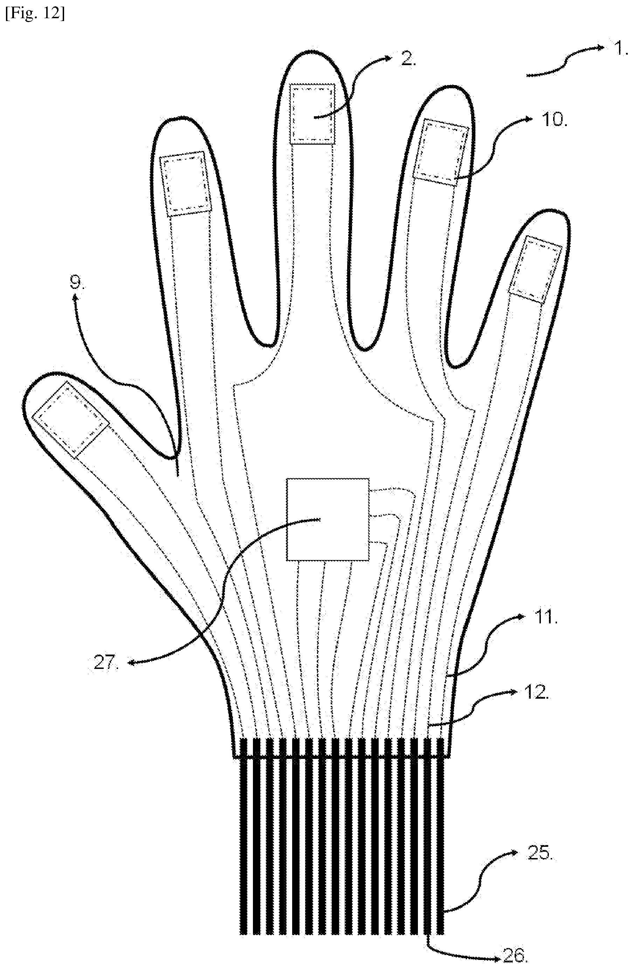

   [Fig.12] illustrates a rectangular array of a plurality of mechanical stimuli sensors arranged on the palm face of the glove;
**Fig.13**

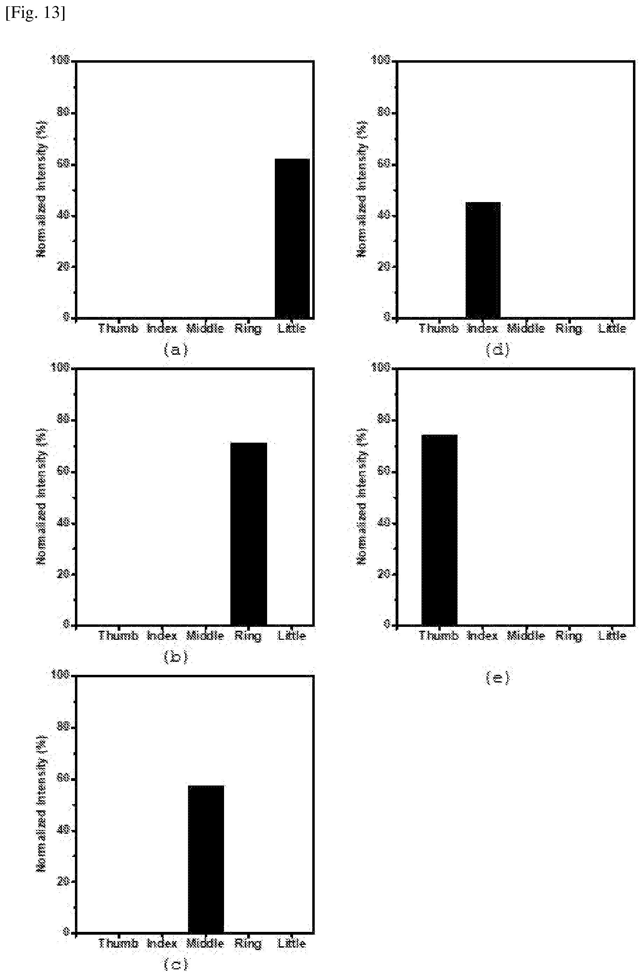

   [Fig.13] illustrates a normalized output of the mechanical stimuli detection glove, in resistive mode, when specific fingers are squeezed;
**Fig.14**

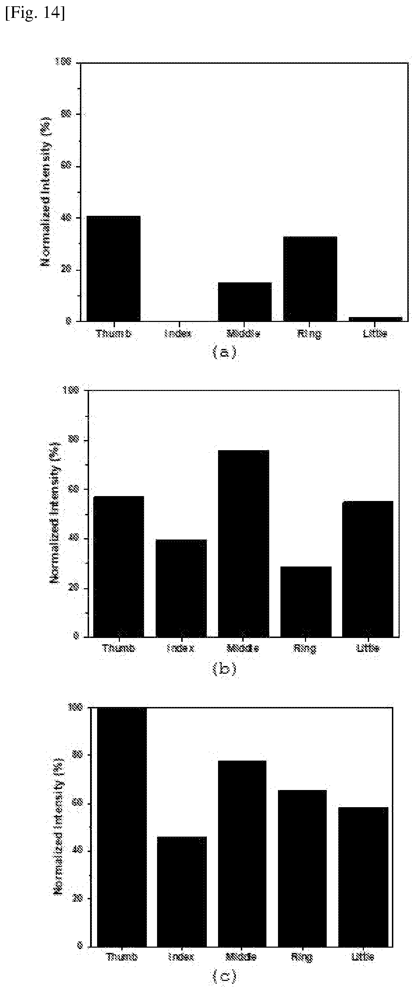

   [Fig.14] illustrates a normalized output of the mechanical stimuli detection glove, in resistive mode, when said glove is holding different objects or when clenching the fist;
**Fig.15**

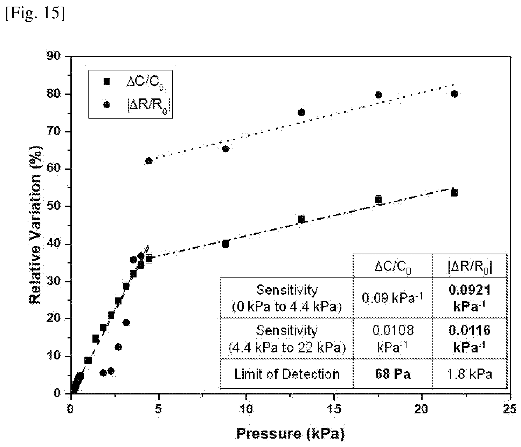

   [Fig.15] illustrates the relative capacitive (ΔC/Cₒ) and resistive changes (ΔR/Rₒ) of a mechanical stimuli sensor for measuring different pressures from 0 kPa to 22 kPa.

### Description of Embodiments

The present invention refers, in a first aspect, to a mechanical stimuli detection glove (1) comprising at least one mechanical stimuli sensor (2) connected to at least one finger zone of said glove (1) wherein said mechanical stimuli sensor (2) comprises:
a first fabric substrate layer (3), which comprises a first layer zone impregnated with a hydrogel (4); and
a second fabric substrate layer (6); and
a first sensor electrode (5) having a portion connected to an upper part of said first layer zone impregnated with a hydrogel (4); and
a second sensor electrode (7) having an upper portion connected to a lower part of said first layer zone impregnated with a hydrogel (4) and a lower portion connected to an upper portion of said second fabric substrate layer (6); wherein
the first sensor electrode (5) is connected to a first conductive line (11); and
the second sensor electrode (7) is connected to a second conductive line (12), and wherein the second fabric substrate layer (6) comprises a second layer zone impregnated with hydrogel (30).

According to the invention, as illustrated in figures 7 or 8, the second fabric substrate layer (6) comprises a second layer zone impregnated with hydrogel (30). This embodiment allows that the feedback to the user be felt at the point of pressure measurement, enabling a natural and intuitive interaction between the user and the mechanical stimuli detection glove.

In another preferred embodiment according to the invention, as illustrated in figures 9 or 10, the mechanical stimuli sensor (2) further comprises:
a third fabric substrate layer (29), which comprises a second layer zone impregnated with a hydrogel (30); and
a third sensor electrode (28) having an upper portion connected to a lower part of the second fabric substrate layer (6) and a lower portion connected to an upper portion of said second layer zone impregnated with a hydrogel (30); wherein
the third sensor electrode (28) is connected to a third conductive line (31).

In a preferred embodiment according to the invention, the mechanical stimuli sensor (2) is connected to at least one of the group consisting of an internal first glove layer (8), an external first glove layer (9) or an internal second glove layer (32) forming a zone for insertion of a finger (23), or an external second glove layer, wherein said mechanical stimuli sensor (2) is arranged at an adjacent position in relation to said zone for insertion of a finger (23), preferably in an end portion of said zone for insertion of a finger (23). As illustrated in figures 1 or 2, the mechanical stimuli sensor (2) is connected to the external first glove layer (9), wherein this embodiment is particularly advantageous because the sensor may enter directly in contact with an object or surface, when it is touched or grasped by the glove, increasing the precision of the results.

Alternatively, as illustrated in figure 5, the mechanical stimuli sensor (2) is connected simultaneously to an internal first glove layer (8) and to an external first glove layer (9), wherein this embodiment allows that the skin of the user does not directly touch the sensor. This configuration avoids mechanical damages or chemical degradations to the electrodes.

In other preferred embodiments, as illustrated in figure 6, the mechanical stimuli sensor (2) is connected simultaneously to an internal first glove layer (8) and to an external first glove layer (9) and said mechanical stimuli sensor (2) is covered by an external second glove layer (21) to protect said sensor from external damages or humidity.

Preferably, the mechanical stimuli sensor (2) is arranged on a distal phalanx zone (24) in relation to the palmar side of said glove (1), considering that this zone is relevant for assessing of touch or pressure sensations.

Preferably, at least one of the respective zones for insertion of a finger (23), namely the thumb finger, the index finger, the middle finger, the ring finger, and the little finger apertures of said glove (1) comprises at least one mechanical stimuli sensor (2) adjacently connected to said respective zones for insertion of a finger (23).

In other embodiments of the mechanical stimuli detection glove (1), a mechanical stimuli sensor (2) is arranged on the distal phalanx zone (24) of the thumb, index, middle, ring and little fingers in relation to the palmar side of said glove (1), as illustrated in figure 1.

Considering the aimed data and results regarding kinesthetic or force feedback or tactile feedback, at least one rectangular array of a plurality of mechanical stimuli sensors (27) may be arranged on the palm face of the glove (1), namely on the middle phalanx, the proximal phalanx, the hypothenar zone or the thenar zone of the glove (1), as illustrated in figure 12. Moreover, a plurality of mechanical stimuli sensor (2) may be arranged on zones of the glove that touch creases of the hand on the palm face, namely the distal interphalangeal the proximal interphalangeal, the palmar digital, the distal palmar, the proximal palmar and the thenar zones, wherein this embodiment is particularly useful to detect bending of the fingers.

A plurality of mechanical stimuli sensor (2) also may be arranged on the back face of the glove (1), in relation to the palm face.

The present invention is used in applications wherein it is necessary to know the force or pressure exerted by the fingers or hand and in applications wherein a feedback signal applied to the fingers or hand is useful. Some applicability examples of the inventions are hand and/or fingers pressure monitoring, haptic response monitoring, monitoring of physiotherapy results for a user, evaluation of improvement regarding performance and/or injuries prevention in sports that rely on hands movements (such as tennis, badminton, paddle, baseball, handball, basketball, korfball, volleyball, athletics, and others), connection with computational devices that provide experiences of virtual/augmented reality, or connection with computational devices that provide human-machine interfaces. In the context of the present invention, haptic response monitoring is referred to creation of an experience of touch by applying forces, vibrations, or motions to the user.

The feedback signal, pertinent to the applications wherein a feedback signal is applied to the fingers or hand, can be an electrostimulation impulse, which is imposed on the fingertip adjacent to the zone for insertion of a finger (23). The electrostimulation impulse comprises the application of a small electrical current through the fingertip. Considering the exemplary embodiment of figure 7, this current is delivered through the second sensor electrode (7) that is connected to second fabric substrate layer (6) arranged on the fingertip, and exits through a ground electrode, for example the second sensor electrode (7) of another mechanical stimuli sensor (2), arranged at an adjacent position in relation to another zone for insertion of a finger (23), namely a mechanical stimuli sensor (2) located on a different finger.

The first sensor electrode (5), the second sensor electrode (7) and the third sensor electrode (28) comprise a conductive material and are sewn or printed on a fabric portion of the glove. The glove comprises a first layer zone impregnated 12 with a hydrogel (4) and can comprise a second layer zone impregnated with a hydrogel (30). The hydrogel surface of said second layer zones impregnated with a hydrogel (30) contacts the fingertip, as shown in figures 8 or 10, since this material presents the ideal electrical properties to establish electrical connection to the fingers. The intensity and duration of the current applied in the fingertip can be controlled by the user or configured into a computing device connected to a mechanical stimuli detection system, which comprises the mechanical stimuli detection glove (1). The purpose of applying an electrostimulation impulse to the fingertip in a glove may be for a variety of reasons, such as to stimulate muscle activity, reduce pain, or improve circulation, or as a user's feedback method. Any therapeutic uses of the glove described here are not claimed.

Additionally, the pressure sensing characteristics of the mechanical stimuli detection glove (1) and the electrostimulation feedback feature could be combined for a symbiotic application. In the situation where an applied pressure by the finger or group of fingers is above a predefined threshold, the electrostimulation feature can be used to provide electrical feedback to the user. Therefore, through the application of an electrical current in the fingertip, the user is informed that the pressure exerted exceeds the safe threshold value and the pressure should be reduced.

As it is illustrated in figure 2, a connector (10), for instance a sewing line or an adhesive, connects the mechanical stimuli sensor (2) to at least a glove layer selected from the group consisting of an internal first glove layer (8) or an external first glove layer (9), an external second glove layer (21) or an internal second glove layer (32). The sewing line or the adhesive are applied on the periphery of the first fabric substrate layer (3) and the second fabric substrate layer (6), with the proviso that the connector (10) does not interfere with the first layer zone impregnated with a hydrogel (4).

Preferably, the mechanical stimuli sensors (2) based on hydrogel are embedded in the glove (1) through a simple sewing process with a regular sewing line. This is possible due to the substrates of the pressure sensors being based on fabric, thus compatible with sewing. By sewing the mechanical stimuli sensors (2) to a regular non-sensitive glove, and given the material of the sensors' substrate, the flexibility of the original glove is maintained, and the glove is endowed with the ability to detect mechanical stimuli. The flexibility of the sensing elements also allows the user to grasp objects with irregular surfaces without a compromise of the pressure sensing, since the sensing elements, together with the glove, can shape up to the object being grasped.

Furthermore, the user will have a native feeling when wearing the glove (1), given that the sensing elements exploit similar materials to those used in the glove (fabrics). Therefore, the user will not feel the presence of a stiff sensing element, thus being able to comfortably wear the glove (1) and make natural movements. The sensitive glove (1) becomes able to distinguish and discriminate distinct activities, namely the grasping of objects with grasping force quantification, hand movements (such as hand opening and closing, hand clenching and fisting, fingers pointing, etc.), amongst others. Moreover, the mechanical stimuli sensors (2) based on the hydrogel can also use the original fabric of the glove as a part of the mechanical sensing device, both for printing or sewing of the first conductive lines (11), the second conductive lines (12), the third conductive lines (31) or for the incorporation of the hydrogel inside the glove fabric in order to produce the first layer zone impregnated with a hydrogel (4) or the second layer zone impregnated with a hydrogel (30).

In other embodiments according to the invention, as illustrated in figure 3, at least a hydrophobic insulating material (22) is deposited in at least one of the group consisting of the first fabric substrate layer (3), the second fabric substrate layer (6), and the third fabric substrate layer (29) to delimit the first layer zone impregnated with a hydrogel (4) or the second layer zone impregnated with hydrogel (30).

Preferably, the hydrophobic insulating material (22) comprises at least one of the group consisting of a non-charged hydrophobic polymer or a polycyclic aromatic hydrocarbon. More preferably, the hydrophobic insulating material (22) comprises at least one of the group consisting of a lipid polymer, a carbohydrate polymer, a modified carbohydrate polymer, a vinyl polymer, a polycyclic aromatic hydrocarbon, mixtures thereof, or, in the case of the recited polymers, copolymers thereof. The hydrophobic insulating materials (22) increases the impermeabilization of water-based materials, namely the first layer zone impregnated with a hydrogel (4), or the second layer zone impregnated with hydrogel (30). More preferably, when the hydrophobic insulating material (22) comprises a lipid polymer, which is a wax and includes organic compounds insoluble in water that are lipophilic and malleable solids at ambient temperature, and present melting points above 40°C.

The hydrophobic insulating materials (22) may be waxes, which are organic compounds insoluble in water that are lipophilic and malleable solids at ambient temperature, and present melting points above 40°C.

In other embodiments, according to the present invention, the palm zone of the glove (1) comprises at least one rectangular array of a plurality of mechanical stimuli sensors (27), which are arranged in rows and columns. The rectangular array is comprised by a plurality of first sensor electrodes (5) and second sensor electrodes (7) and a plurality of first layer zones impregnated with a hydrogel (4), which is configured for the electrical detection of different mechanical stimuli, such as pressure, touch, strain, or elongation. The electrical connection in series of different first sensor electrodes (5) and second sensor electrodes (7) in either rows or columns enables the formation of an array configuration of several mechanical stimuli sensors (2) comprising a first zone impregnated with a hydrogel (4). This planar (XY) structure enables the detection of the exact position of the mechanical stimuli on the surface of the palm of the glove. Preferably, said rows or columns are arranged orthogonally among themselves, wherein each row or column comprises a plurality of mechanical stimuli sensors (2). In the preferred embodiments of the invention, the rectangular array of a plurality of mechanical stimuli sensors (27) also comprises a plurality of third sensor electrodes (28), and of second layer zones impregnated with a hydrogel (30).

The hydrogel is a piezo-responsive material, which can be defined as a three-dimensional (3D) network of hydrophilic polymers that can swell in water and retain a large amount of water while maintaining its structure, due to chemical or physical cross-linking of individual polymer chains.

In the preferred embodiments according to the present invention, the first layer zone impregnated with a hydrogel (4) or the second layer zone impregnated with hydrogel (30) includes a hydrogel selected from the group consisting of a cellulose derivative, a polynucleotide, a polypeptide, a polysaccharide, a natural rubber, a polyphenolic polymer, a polyacrylamide, a complex of polymers of large chain fatty acids or their mixtures or their composites.

Several hydrogels may be selected according to their sustainable features regarding recycling or compostability, namely hydrogels comprising cellulose-based biopolymers.

In the most preferred embodiments, the hydrogel is a cellulose derivative hydrogel. More preferably, the cellulose derivative hydrogel is selected from the group consisting of cellulose acetate, cellulose acetate phthalate, cellulose acetate butyrate, cellulose acetate trimellitate, hydroxypropyl methylcellulose phthalate, methylcellulose, ethyl cellulose, hydroxyethyl cellulose, carboxymethyl cellulose, sodium carboxymethyl cellulose, hydroxypropyl cellulose, hydroxypropyl methylcellulose, their composites or their composites with natural polymers, polyvinyl alcohol, polyelectrolyte complexes, interpenetrating polymer network, cellulose-inorganic hybrid hydrogels or their mixtures or their composites. In the most preferred embodiments, the cellulose derivative hydrogel is carboxymethyl cellulose or sodium carboxymethyl cellulose.

In the preferred embodiments according to the invention, the first layer zone impregnated with a hydrogel (4), or the second layer zone impregnated with hydrogel (30) comprise the cellulose derivative hydrogel in a mass percentage from about 0.01% to about 50.00% in relation to the overall mass of said first layer zone impregnated with a hydrogel (4) or said second layer zone impregnated with hydrogel (30). In the most preferably embodiments, the cellulose derivative hydrogel, for example the sodium carboxymethyl cellulose, carboxymethyl cellulose, or the carboxyethyl cellulose is in a mass percentage from about 0.01% to about 10.00% in relation to the overall mass of said the layer zone impregnated with a hydrogel (4) or said second layer zone impregnated with hydrogel (30).

Preferably, the mass ratio between water and the cellulose derivative hydrogel in the first layer zone impregnated with a hydrogel (4) or the second layer zone impregnated with hydrogel (30) is in the range from about 0.01 to about 1000, more preferably said mass ratio is in the range from 1 to 100.

The hydrogels, as described above, are formed through a chemical or physical cross-linking of individual polymer chains. The chemical crosslinking can be achieved when the polymer is combined through a chemical reaction with ionic salts composed of an anion (mono, di or trivalent) and a cation (mono, di or trivalent). Other forms of chemical cross-linking are also possible, as covalent crosslinking, as it will be understood by a person skilled in the art. In the preferred embodiments of the present invention, the crosslinking agent includes at least one of the group consisting of an ionic crosslinking agent or a covalent crosslinking agent. The crosslinking agent also contributes to the mechanical stability and integrity of the first layer zone impregnated with a hydrogel (4) or the second layer zone impregnated with hydrogel (30).

In the preferred embodiments according to the present invention, at least one of the first layer zone impregnated with a hydrogel (4) or the second layer zone impregnated with hydrogel (30) includes at least a salt in the hydrogel matrix, wherein said salt includes a cation selected from a group consisting of a monovalent cation, a divalent cation, or a trivalent cation. Preferably, the divalent or trivalent cations are used as ionic-crosslinkers.

Preferably, the first layer zone impregnated with a hydrogel (4), or the second layer zone impregnated with hydrogel (30) comprise at least a salt selected from a group consisting of a nitrogen quaternary salt, a urea derivative salt, a salt of general formula MₓA_{y} or their mixtures; and wherein M is a cation selected from a group consisting of Na, Li, K, Be, Mg, Ca, Ba, Zn, Ni, Cu, Al, Fe⁺², Fe⁺³; wherein A is an anion selected from a group consisting of F, Cl, Br, I, hydroxide, sulfate, phosphate, carboxylate, carbonate, tosylate, nitrate, acetate, thiocyanate, methanoate, tetrafluoroborate, dicyanamine, tretrafluoroborate, hydrogensulfate, methylsulfonate, tricyanamide, trilfuoromethane-sulfonate, bis(trifluoromethyl)azanide, ethylsulfate, benzoate, tetracyanoborate, salicylate, methoxyethylsulfate, aluminiumtetracholate; wherein x and y are independently integers of value equal or superior to 1 selected to provide the valence of the cation M according to the valence of the combined anion A.

Regarding the salts recited in the previous paragraph, the cations are preferably zinc, calcium, magnesium, nickel, or copper cations. The most preferred salts employed in the present invention are zinc or calcium, which are classified as non-toxic and non-critical materials regarding environmental issues. The most preferred anion employed in said salts is chloride.

Preferably, the mass ratio between the cellulose derivative hydrogel and the salt in said first layer zone impregnated with a hydrogel (4) or said second layer zone impregnated with hydrogel (30) is in the range from 0.0001 to 10000.

Preferably, the first layer zone impregnated with a hydrogel (4), or the second layer zone impregnated with hydrogel (30) comprise the ionic crosslinking agent in a concentration from about 0.01 M to about 50.00 M in relation to the mass of water in said first layer zone impregnated with a hydrogel (4) or said second layer zone impregnated with hydrogel (30).

When the crosslinking agent comprises a covalent crosslinking agent, it is preferably selected from a group consisting of epoxy resins such as epichlorohydrin, dicarboxylic acids such as citric acid, oxalic acid, dialdehydes such as glutaraldehyde, glyoxal, polyethylene glycol dialdehyde diethyl acetal, aldehydides formaldehyde, hemi acetals such as genipin, acrylamides such as N,N'-methylenebisacrylamide, telechelic poly(vinyl alcohol), boron salts such as borax, N-hydroxysuccinimide esters, divinyl compounds such as divinyl sulfone or their mixtures. Preferably, the mass ratio between the covalent crosslinking agent and the cellulose derivative hydrogel in in said first layer zone impregnated with a hydrogel (4) or said second layer zone impregnated with hydrogel (30) is in the range from about 0.01 to about 100.00.

Preferably, the mass ratio between the covalent crosslinking agent and the cellulose derivative hydrogel in said first layer zone impregnated with a hydrogel (4) or said second layer zone impregnated with hydrogel (30) is in the range from 0.01 to 100.00.

Without willing to be bonded by any theory whatsoever, a proper concentration of the ions referred to the salts incorporated in the first layer zone impregnated with a hydrogel (4) or in the second layer zone impregnated with hydrogel (30) makes possible to perform resistance and capacitance measurements, because occurs an effective electrical current through said hydrogels layers, considering the water incorporated in the hydrogel matrix, which allows free movements of the ions in the matrix.

The cellulose-based hydrogels present a mechanical response to deformations due to two different properties: i) they are easily deformable when external forces are applied, and ii) the presence of different cations in the structure of the cellulose hydrogels creates percolation paths of electrical conduction in said materials. Therefore, when the hydrogel is subjected to pressure or strain, these materials can be used as active layers in a mechanical stimuli sensor. The sensing method can be based on the measurement of intrinsic changes in resistance (continuous current), impedance (alternate current), capacitance of the hydrogel, or both, as well as changes in voltage or current of an electrical signal applied to the hydrogel. The electrical measurements of these changes can be further achieved in an array or matrix configuration of a plurality of mechanical stimuli sensors (2). This planar (XY) structure enables the detection of the exact position of the mechanical stimulus on the surface of the hydrogel.

Furthermore, it is possible to enhance the performance of the sensors when operating alternately in the capacitive and resistive modes by adding ions to the first layer zone impregnated with a hydrogel (4). Combining capacitive and resistive properties in the first layer zone impregnated with a hydrogel (4) allows the mechanical stimuli sensor (2) to detect lower intensity mechanical stimuli and present an increased resolution for several intensity of mechanical stimuli. This embodiment overcomes the problems of prior art referred to limiting of the native feeling by the user when capacitive and resistive sensors demand a specific layer for incorporation of the resistive elements, and another specific layer for incorporation of the capacitive elements. These stacked sensitive double layers increase the thickness of the sensorial layers and impair the native feeling.

The electrical measurements of these changes must be achieved with the incorporation of electrodes, establishing a mechanical sensing element composed of the first layer zone impregnated with a hydrogel (4) with two different electrodes on the top and bottom surfaces, namely at least a first sensor electrode (5) on the first fabric substrate layer (3) and at least a second sensor electrode (7) on the second fabric substrate layer (6), due to the impregnation feature of the hydrogel in the first fabric substrate layer (3).

In the preferred embodiments according to the present invention, at least one of the first sensor electrode (5), the second sensor electrode (7), the third sensor electrode (28), the first conductive line (11), the second conductive line (12) or the third conductive line (31) is made of a material selected from the group consisting of an electrically conductive material, such as carbon, silver, gold, platinum, copper, aluminum, alloys thereof, or metallic alloys, for example stainless steel; or a conductive polymer or copolymer, for example polyaniline or poly (3,4-ethylenedioxythiophene) polystyrene sulfonate (PEDOT:PSS). These conductive materials ensure electrical connection between elements.

In the preferred embodiments according to the present invention, at least one of the group consisting of the first sensor electrode (5), the second sensor electrode (7) or the third sensor electrode (31) is selected from the group consisting of a ribbon, a strip or a wire.

Conductive lines are sewn or printed on the first fabric substrate layer (3) and on the second fabric substrate layer (6) to allow the connection between the first sensor electrode (5) and the second sensor electrode (7) to the conventional electronics for data acquisition and transmission. Moreover, and with the same goal, conductive lines are sewn or printed on the third fabric substrate layer (29).

To avoid short circuits between both first sensor electrode (5) and the second sensor electrode (7), the electrodes overlap only on the area with the first layer zone impregnated with a hydrogel (4), extending for five to ten millimeters for one of the sides of the overlapping area. The conductive lines, namely the first conductive line (11) or the second conductive line (12) are then sewn or printed over said extension, and their length is some centimeters (from ten to twenty centimeters), allowing them to reach the wrist of the hand, and thus the end of the mechanical stimuli detection glove (1). Regular conductive cables, namely the first conductive cable (25) or the second conductive cable (26), can then be welded to the extremity of the conductive sewn or printed lines, for example silver lines, to establish the connection with conventional electronics.

The step of depositing and impregnating a hydrogel in a first fabric substrate layer (3) or in a third fabric substrate layer (29), respectively forming at least a first layer zone impregnated with a hydrogel (4) or at least a second layer zone impregnated with a hydrogel (30), can be carried out to form a pattern in specific regions of the first fabric substrate layer (3), of the second fabric substrate layer (6), or of the third fabric substrate layer (29) to create individualized pixels.

The first conductive line (11), the second conductive line (12) and the third conductive lines (31) are selected from the group consisting of an electrically conductive material, such as carbon, silver, gold, platinum, copper, aluminum, alloys thereof, or metallic alloys, for example stainless steel, either in the form of wires, threads, or inks; or a conductive polymer or copolymer, for example polyaniline or poly (3,4-ethylenedioxythiophene) polystyrene sulfonate (PEDOT:PSS). The first conductive cable (25), the second conductive cable (26) and the third conductive cable are coated by an insulant cover as it will be understood by a person skilled in the art. Preferably, the first conductive lines (11), the second conductive line (12), the third conductive lines (31), the first conductive cable (25), the second conductive cable (26) or the third conductive cable are arranged inside of mechanical stimuli detection glove, for example arranged between two fabric substrate layers, more preferably between the internal first glove layer (8) and the external first glove layer (9), as it is illustrated in figure 1.

Preferably, in the mechanical stimuli detection glove (1), a first conductive cable (25) is connected to the first conductive lines (11), a second conductive cable (26) is connected to the second conductive lines (12), and a third conductive cable is connected to the third conductive lines (31).

The plurality of first conductive cables (25), second conductive cables (26) and the third conductive cables are preferably exposed at the wrist zone of the glove (1), as it is illustrated in figure 1, or at a forearm zone of the glove (1), wherein said plurality of cables may be put together by a connector and be linked to a hybrid connector (13), in order to send the analog signals retrieved by the sensors to said hybrid connector (13).

In the preferred embodiments of the mechanical stimuli detection glove (1), any one of the first fabric substrate layer (3), the second fabric substrate layer (6), the internal first glove layer (8), the external first glove layer (9), the external second glove layer (21), the third fabric substrate (29) or the internal second glove layer (32) is selected from a group consisting of a fibrous insulator woven fabric or a fibrous insulator unwoven fabric, for example a cotton fabric. More preferably, the first fabric substrate layer (3), the second fabric substrate layer (6) and the third fabric substrate layer (29) are selected from a group consisting of a fibrous insulator woven fabric or a fibrous insulator unwoven fabric. The first fabric substrate layer (3), the second fabric substrate layer (6), and the third fabric substrate layer (29) can be made of natural fibers, synthetic fiber, and mixtures thereof. More preferably the natural fibers are selected from a group consisting of cotton fibers, wool fibers, silk fibers, flax fibers or mixtures thereof. More preferably the synthetic fibers are selected from a group consisting of polyamide fibers, polyester fibers, acrylic fibers, polyolefin fibers, polyether-polyurea copolymers, for example lycra, or mixtures thereof.

The internal first glove layer (8), the external first glove layer (9), the external second glove layer (21) or the third fabric substrate layer (29) are made of other suitable material for gloves productions. As examples of suitable materials, are recited the different polymers including natural rubber latex, nitrile butadiene rubber, polyvinyl chloride or neoprene or their copolymers.

The present invention refers in second aspect, to a method of preparation of a mechanical stimuli detection glove (1) comprising the following steps regarding the preparation of a mechanical stimuli sensor (2):
a) Depositing and impregnating a hydrogel in a first fabric substrate layer (3) forming a first layer zone impregnated with a hydrogel (4);
b) Connecting a portion of a first sensor electrode (5) to an upper part of said layer zone impregnated with a hydrogel (4);
c) Connecting a portion of a second sensor electrode (7) to a lower part of said first layer zone impregnated with a hydrogel (4) and to an upper portion of a second fabric substrate layer (6);
d) Connecting a first conductive line (11) to the first sensor electrode (5);
e) Connecting a second conductive line (12) to the second sensor electrode (7);
f) Connecting the mechanical stimuli sensor (2) to at least one finger zone of said glove (1);
wherein the steps b) and c) are executed in any order between them, and the steps d) and e) are executed in any order between them.

In the preferred embodiments, the method of preparation of a mechanical stimuli detection glove (1) further comprises, before the step f), the following steps:
i) Depositing and impregnating a hydrogel in a second fabric substrate layer (6) forming a second layer zone impregnated with a hydrogel (30);
ii) Connecting a portion of the second sensor electrode (7) to an upper part of said second layer zone impregnated with a hydrogel (30).

In other preferred embodiments, the method of preparation of a mechanical stimuli detection glove (1) further comprises, before the step f), the following steps:
i) Depositing and impregnating a hydrogel in a third fabric substrate layer (29) forming a second layer zone impregnated with a hydrogel (30);
ii) Connecting a portion of a third sensor electrode (31) to an upper part of said second layer zone impregnated with a hydrogel (30);
iii) Connecting a portion of the third sensor electrode (31) to a lower part of said second fabric substrate layer (6).

In the preferred embodiments, the method of preparation of a mechanical stimuli detection glove (1) further comprises a step of connecting the mechanical stimuli sensor (2) to at least one of the group consisting of an internal first glove layer (8), an external first glove layer (9) or an internal second glove layer (32) forming a zone for insertion of a finger (23), and said mechanical stimuli sensor (2) being arranged at an adjacent position in relation to said zone for insertion of a finger (23), preferably in an end portion of said zone for insertion of a finger (23). More preferably, said method further comprises a step of covering the mechanical stimuli sensor (2) by an external second glove layer (21).

The method of preparation of a mechanical stimuli detection glove (1) also may comprise a step of depositing a hydrophobic insulating material (22) in at least one of the group consisting of the first fabric substrate layer (3), the second fabric substrate layer (6) or the third fabric substrate layer (29) to delimit the first layer zone impregnated with a hydrogel (4) or the second layer zone impregnated with a hydrogel (30).

The preferred method of preparation of the mechanical stimuli sensor (2) according to the present invention is centered on printing or drop-on-demand methods. This enables a large-scale production of the mechanical stimuli sensor (2), with the possibility to deposit a hydrogel on various fabric substrates, for example cotton fabrics, while maintaining reduced costs of production. The drop-on-demand methods are compatible with large areas.

In other embodiments of preparation of the mechanical stimuli sensor (2) according to the present invention, it is possible to carry out the deposition step by a film application step, for example a Doctor blade; by a screen printing step; by a flexography step; by a spray-coating step; or by an inkjet, a Roll-to-Roll (R2R) compatible, step, as it will be understood by a person skilled in the art. Several methods that can be employed in the deposition step are described in Johanna Zikulnig, Jürgen Kosel, "Flexible Printed Sensors-Overview of Fabrication Technologies", Reference Module in Biomedical Sciences, Elsevier, 2021.

In the most preferred embodiments according to the present invention, the hydrogel is incorporated and impregnated in the first fabric substrate layer (3), in the second fabric substrate layer (6), or in the third fabric substrate layer (29) using thermal treatments below 100 °C to produce, respectively, a first active layer zone impregnated with a hydrogel (4) or a second active layer zone impregnated with a hydrogel (30).

Therefore, the use of a fabric-based fibrous first fabric substrate layer (3), a second fabric substrate layer (6) or a third fabric substrate layer (29) allows the impregnation of other materials, like the biopolymer hydrogels, inside the substrate's structure. In this impregnation method, the porous substrate composed of fibers or treads will be surrounded by the polymeric component throughout all the substrate's thickness.

The method of preparation according to the present invention also enables the individualization of the hydrogel, as the polymeric component can be deposited only at selected and defined regions of the first fabric substrate layer (3), the second fabric substrate layer (6) or the third fabric substrate layer (29). This procedure will create electrically conductive regions on the first fabric substrate layer (3), the second fabric substrate layer (6) or the third fabric substrate layer (29) in a transversal direction to the substrate surface. Therefore, paths for electrical conduction will be formed from the top surface of the first fabric substrate layer (3), the second fabric substrate layer (6) or the third fabric substrate layer (29) to the bottom one, enabling the deposition of electrodes on both sides of said substrates. Moreover, the deposition and impregnation of an electrical insulating material in the substrate can also be established in selected regions to improve the patterning of the hydrogel and to reduce ionic conductivity of natural fibers substrates. Said insulating materials can be non-charged hydrophobic polymers, either synthetic or bio-based, which means poly lipidic-based structures, modified carbohydrate polymers, poly vinylic or poly aromatic hydrocarbons to increase the impermeabilization of water-based materials. More specifically, said insulating materials may be waxes, which are organic compounds insoluble in water that are lipophilic and malleable solids at ambient temperature, and present melting points above 40°C.

Additionally, the impregnation of the hydrogel on the first fabric substrate layer (3), on the second fabric substrate layer (6) or on the third fabric substrate layer (29) creates a symbiotic phenomenon to the mechanical stimuli detection, as said fabric substrate layers fibrous structure provides mechanical stability to the hydrogel formed inside it.

The present invention refers, in a third aspect, as illustrated in figure 11, to a mechanical stimuli detection system comprising the mechanical stimuli detection glove (1), as defined in the first aspect of the invention, and further comprising:
a hybrid connector (13), which is connected to a plurality of first conductive lines (11) and to a plurality of second conductive lines (12) of said glove (1); and
a computing device (15), which is connected to said hybrid connector (13) by a computer connection cable (14);
wherein said computing device (15) comprises a microcontroller (16), which is configured to process data retrieved by the mechanical stimuli sensor (2) and transmits processed data to a data transmission and reception unit (19).

In a preferred embodiment, the mechanical stimuli detection system, according to the third aspect of the invention, comprises a feedback element, which is a second layer zone impregnated with hydrogel (30) comprised in the second fabric substrate layer (6).

In an alternative preferred embodiment, the mechanical stimuli detection system, according to the third aspect of the invention, comprises a feedback element, which is a second layer zone impregnated with hydrogel (30) comprised in a third fabric substrate layer (29), and a third sensor electrode (28) having an upper portion connected to a lower part of the second fabric substrate layer (6) and a lower portion connected to an upper portion of said second layer zone impregnated with a hydrogel (30); wherein the third sensor electrode (28) is connected to a plurality of third conductive lines (31).

Preferably, the power supply (20) comprises at least one of the group consisting of a charging unit (18) or a battery.

All mechanical interaction with the mechanical stimuli detection system can be digitized and visualized in real time on a computer software interface. The mechanical interactions assume the use of hands or fingers, which can serve as a means of interaction between the user and the mechanical stimuli detection glove (1). Subsequently, the digital content can be programmed through an accessible language, especially graphics, allowing to hand and finger pose estimation and motion tracking, through the computer program. Other suitable applications are related to electrostimulation feedback.

The hybrid connector (13) links electrically the mechanical stimuli detection glove (1) to the computing device (15). The hybrid connector (13) can include elements as needles, springs, clamps, alligator clips, or surface electrodes, which can be composed of conductive materials as metals, such as gold, silver, aluminum or copper.

The present invention refers, in a fourth aspect, to a use of a mechanical stimuli detection system, as defined in the third aspect of the invention, as a mechanical stimuli monitoring system based on at least a feedback signal, which is applied to at least one element selected from the group consisting of a finger or a palm hand, wherein said feedback signal is an electrostimulation impulse imposed adjacently to a mechanical stimuli sensor (2) of the mechanical stimuli detection glove (1), as defined in the first aspect of the invention.

Preferably, in said use according to the fourth aspect, the electrostimulation impulse is an electrical current having its input through one component of a conductive group consisting of a second sensor electrode (7) or a third sensor electrode (28) and having its output through another component of said conductive group. More preferably, said use is related to at least one of the group consisting of a hand and/or fingers pressure monitoring, a haptic response monitoring, a monitoring of physiotherapy results for a user, an evaluation of an improvement regarding performance and/or injuries prevention in sports, a connection with computational devices that provide experiences of virtual/augmented reality, or a connection with computational devices that provide human-machine interfaces, wherein these later applications include computational devices that run electronic games. Any therapeutic uses of the glove described here are not claimed.

### Examples

### Example 1

An exemplary formulation of the hydrogel to be used in the method of preparation of a mechanical stimuli detection glove (1) comprises:
- Sodium carboxymethyl cellulose - CMC (Sigma Aldrich Mw approximately 250,000): Solution concentration from about 0.1 % to about 10.0 % weight in water, more preferably from about 1 % to about 5 % weight in water;
- Calcium Chloride (CaCl₂) - Concentration from about 0.01 M to about 10.0 M in water, more preferably from about 1 M to about 5 M in water;
- Zinc Chloride (ZnCl₂) - Concentration from about 0.5 M to about 4.0 M in water. in water, more preferably from about 0.5 M to about 2 M in water.

In the preferred embodiments, elastomers are added to the fibrous substrate layer to improve its mechanical properties, such as polyethylene glycol (PEG) or glycerol.

The hydrogel is deposited and impregnated in a first fabric substrate layer (3), in a second fabric substrate layer (6), or in a third fabric substrate layer (29) forming, respectively, at least a first layer zone impregnated with a hydrogel (4) or a second layer zone impregnated with a hydrogel (30), which may be in continuous form or patterned in specific regions to create individualized pixels. The deposition of the cross-linked hydrogel is carried out by a film applicator (Doctor blade) and by drop-casting (large area compatible). The deposition may also be performed by a reactive two-step deposition of separate hydrogel components (CMC and salts) by drop-on-demand techniques, such as drop-casting, screen printing, spray, or inkjet (R2R compatible).

A cotton fabric, having above 100 thread count, which refers to the total number of threads per square inch, is the substrate as physical support material, as well as the first fabric substrate layer (3), the second fabric substrate layer (6), or the third fabric substrate layer (29) for the impregnation of the hydrogel, and the second fabric substrate layer (6).

The first conductive line (11), the second conductive line (12) and the third conductive line (31) are made of conductive materials wires or threads, for example metals, alloys, preferably stainless steel, carbon or composites thereof. Examples of metals that may be used as conductive lines are Ag, Cu, or Al, preferably Ag. Another preferred embodiment for said conductive lines is a composite of Ag and carbon.

Carbon is often used in the field of printing technologies and may comprise at least one carbon-based material, for example carbon nanotubes, graphene, carbon black or graphite.

The layers of the mechanical stimuli sensor (2) are assembled by sewing with a regular sewing thread. Alternatively, the layers may be assembled using "bio-based glue" or liquid adhesives and by an interlining step employing tapes having a predetermined thickness.

A mechanical stimuli detection sensor incorporated in a mechanical stimuli detection glove according to the present invention was prepared according to the following specifications:
Total sensible area: 11.25 cm²;
Number of pixels including second layer zones impregnated with a hydrogel (30): 1;
Area of each pixel: 2.25 cm²;
Minimum distance between pixels: 0.9 cm;
Nominal pixel resistance: 10 kΩ to 10 MΩ;
Sensitivity: 10⁻¹ kPa⁻¹ (below 4.4 kPa) / 10⁻² kPa⁻¹ (above 4.4 kPa);
Limit of detection: 1 g;
Response time: < 1 s;
Relaxing time: < 10 s;

### Example 2

The functioning of the mechanical stimuli detection system is further exemplified in the following paragraphs. To acquire data from the mechanical stimuli detection glove (1), each mechanical stimuli sensor (2) is connected in series to an auxiliary resistor of variable value, ideally matching the internal resistance of the mechanical stimuli sensor (2) when no pressure is applied to it or there is an absence of mechanical stimuli.

Then, a Direct Current (DC) voltage signal of approximately 5 V is applied to the mechanical stimuli sensor (2) in series with the auxiliary resistor. The output voltage of the mechanical stimuli sensor (2) is measured and converted into analog-to-digital converter (ADC) values, which vary between 0 ADC value, corresponding to the lowest resistance value achievable by the mechanical stimuli sensor (2) (which occurs at the maximum pressure), and 1023 ADC value, corresponding to the highest resistance value achievable by the mechanical stimuli sensor (2) (which occurs at the absence of mechanical stimuli).

The acquired data is then processed by a microcontroller (16) comprised in a computing device (15).

As an example, designed to illustrate the acquirement of capacitive data from the mechanical stimuli detection glove (1), each mechanical stimuli sensor (2) is connected in series to an auxiliary resistor of variable value, ideally matching the internal resistance of the mechanical stimuli sensor (2), when no pressure is applied to it or there is an absence of mechanical stimuli. Also, the electrical circuit to measure capacitance can have an extra resistor, smaller in value and, ideally, should not exceed 500 Ohms. Then, a Direct Current (DC) voltage signal of 5 V is applied to the mechanical stimuli sensor (2) in series with an auxiliary resistor. While this voltage is applied, the smaller resistor can be connected to a high-impedance end. This allows the mechanical stimuli sensor (2) to charge by avoiding a significant amount of current to flow through that resistor. This state is maintained until the voltage across the mechanical stimuli sensor (2) reaches a threshold that is equal to 63.2 % of the input voltage signal. The voltage across the mechanical stimuli sensor (2) is continually checked by the ADC. Once the threshold is achieved, the input signal changes its state to high impedance. The smaller resistor is then connected to a low-impedance end, enabling the current to flow through that resistor, and consequently, discharging the mechanical stimuli sensor (2). The time measured between the applied input voltage signal and the achieved threshold is known as Resistance Capacitance (RC) time constant. This time constant defines the various levels of capacitance and therefore pressure, where a low RC time constant indicates a low level of pressure, and a high RC time constant is a result of a high pressure.

As an example, designed to illustrate a semi-quantitative pressure detection by the mechanical stimuli detection glove (1), the plurality of mechanical stimuli sensors (2) go through two calibrations. In the first calibration, in the absence of mechanical stimuli, the ADC values of the plurality of mechanical stimuli sensors (2) are acquired for 5 seconds and averaged. These average values are then subtracted to the following measured values, so that, in the absence of mechanical stimuli, the normalized output signal corresponds to 0 %. In the second calibration, when clenching the fist with the maximum force that the user can exert, the ADC values of the plurality of mechanical stimuli sensors (2) are acquired for 5 seconds and averaged. These average values are then subtracted to the following measured values, so that, in the presence of an extreme mechanical stimuli, the normalized output signal corresponds to 100 %. After calibration, all the ADC values measured fall within the range from 0 % to 100 %. This calibration can, however, be adapted so that the minimum and maximum mechanical stimulus correspond to other situations of interest. The final normalized output signal can also be adjusted to other values of interest.

The figure 13 illustrates: a normalized intensity in percentage output of the mechanical stimuli detection glove (1), in resistive mode, when only the little finger is squeezed (a); when only the ring finger is squeezed (b); when only the middle finger is squeezed (c); when only the index finger is squeezed (d); and when only the thumb finger is squeezed (e).

The figure 14 illustrates: a normalized intensity in percentage output of mechanical stimuli detection glove (1), in resistive mode, when said glove is holding an empty paper cup (very light object) (a); when said glove is holding a full glass bottle (very heavy object) (b); and when clenching the fist (c).

The figure 15 illustrates: the relative capacitive (ΔC/Cₒ) and resistive changes (ΔR/Rₒ) of a mechanical stimuli sensor, prepared according to example 1, for measuring different pressures from 0 kPa to 22 kPa. The capacitive mode operation of the sensor allows it to detect lower pressure values up to a limit of detection of 68 Pa. The resistive mode operation of the sensor allows it to detect higher pressure values up to a limit of detection of 1.8 KPa. Moreover, it is possible to observe that the sensitivity in both operation modes is substantially similar.

As used in this description, the expressions "about" and "approximately" refer to a range in values of roughly 10% the specified number.

As used in this description, the expression. "substantially" means that the real value is within an interval of about 10% of the desired value, variable or related limit, particularly within about 5% of the desired value, variable or related limit or particularly within about 1% of the desired value, variable or related limit.

Moreover, the term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A; X employs B; or X employs both A and B.

In addition, the articles "a" and "an" as used in this application and the appended claims should generally be construed to mean "one or more" unless specified otherwise or clear from the context to be directed to a singular form.

Further, as used herein, the term "exemplary" is intended to mean serving as an illustration or example of something and is not intended to indicate a preference.

The subject matter described above is provided as an illustration of the present invention and must not be interpreted to limit it. The terminology used with the purpose of describing specific embodiments, according to the present invention, must not be interpreted to limit the invention. As used in this description, the definite and indefinite articles, in their singular form, aim to include in the interpretation the plural forms, unless the context of the description explicitly indicates the contrary. It will be understood that the expressions "comprise" and "include", when used in this description, specify the presence of the characteristics, the elements, the components, the steps and the related operations, but do not exclude the possibility of other characteristics, elements, components, steps and operations from being also contemplated.

All modifications, providing that they do not modify the essential features of the following claims, must be considered within the scope of protection of the present invention.

### Reference Signs List

- 1.: a mechanical stimuli detection glove
- 2.: a mechanical stimuli sensor
- 3.: a first fabric substrate layer;
- 4.: a first layer zone impregnated with a hydrogel;
- 5.: a first sensor electrode;
- 6.: a second fabric substrate layer;
- 7.: a second sensor electrode;
- 8.: an internal first glove layer;
- 9.: an external first glove layer;

- 10.: a connector between the mechanical stimuli sensor and a glove layer (a sewing line or an adhesive);
- 11.: a first conductive line;
- 12.: a second conductive line;
- 13.: a hybrid connector;
- 14.: a computer connection cable;
- 15.: a computing device;
- 16.: a microcontroller;
- 17.: a remote communication unit;
- 18.: a charging unit;
- 19.: a data transmission and reception unit;
- 20.: a power supply;
- 21.: an external second glove layer;
- 22.: a hydrophobic insulating material;
- 23.: a zone for insertion of a finger;
- 24.: a distal phalanx zone;
- 25.: a first conductive cable;
- 26.: a second conductive cable;
- 27.: a rectangular array of a plurality of mechanical stimuli sensors;
- 28.: a third sensor electrode;
- 29.: a third fabric substrate layer;
- 30.: a second layer zone impregnated with hydrogel;
- 31.: a third conductive line;
- 32.: an internal second glove layer.

### Patent Literature

PTL1: Chinese patent application CN110313663A of Ningbo Renhe Tech CO LTD., entitled "Smart gloves" and published on October 11th, 2019.
PTL2: South-Korean patent application KR20180117890A of LG Innotek CO LTD, entitled "GLOVE OF SENSING PRESSURE" and published on October 30th, 2018.
PTL3: North American patent US2014215684A1 of Hardy Timothy J and Datyner Nicholas G, entitled "Pressure Sensing Glove" and published on August 7th, 2014.

Chinese patent application CN106445168A of Univ. Central South, entitled "Intelligent gloves and using method thereof" and published on February 2nd, 2017.

North American patent US20200319039A1 of The Regents of the University of California, entitled "Supercapacitive iontronic nanofabric sensing" and published on October 8th, 2020.

### Non Patent Literature

NPL1: Jia-wen Zhang, Yan Zhang, Yuan-yuan Li & Ping Wang (2022) Textile-Based Flexible Pressure Sensors: A Review, Polymer Reviews, 62:1, 65-94, DOI: 10.1080/15583724.2021.1901737

## Claims

1. A mechanical stimuli detection glove (1) comprising at least one mechanical stimuli sensor (2) connected to at least one finger zone of said glove (1) wherein said mechanical stimuli sensor (2) comprises:
a first fabric substrate layer (3), which comprises a first layer zone impregnated with a hydrogel (4); and
a second fabric substrate layer (6); and
a first sensor electrode (5) having a portion connected to an upper part of said first layer zone impregnated with a hydrogel (4); and
a second sensor electrode (7) having an upper portion connected to a lower part of said first layer zone impregnated with a hydrogel (4) and a lower portion connected to an upper portion of said second fabric substrate layer (6); wherein
the first sensor electrode (5) is connected to a first conductive line (11); and
the second sensor electrode (7) is connected to a second conductive line (12); **characterized in that** the second fabric substrate layer (6) comprises a second layer zone impregnated with hydrogel (30).

2. The mechanical stimuli detection glove (1), according to claim 1, wherein further comprises:
a third fabric substrate layer (29), which comprises a second layer zone impregnated with a hydrogel (30); and
a third sensor electrode (28) having an upper portion connected to a lower part of the second fabric substrate layer (6) and a lower portion connected to an upper portion of said second layer zone impregnated with a hydrogel (30); wherein
the third sensor electrode (28) is connected to a third conductive line (31).

3. The mechanical stimuli detection glove (1), according to any of the previous claims, wherein the mechanical stimuli sensor (2) is connected to at least one of the group consisting of an internal first glove layer (8), an external first glove layer (9) or an internal second glove layer (32) forming a zone for insertion of a finger (23), wherein said mechanical stimuli sensor (2) is arranged at an adjacent position in relation to said zone for insertion of a finger (23), preferably in an end portion of said zone for insertion of a finger (23).

4. The mechanical stimuli detection glove (1), according to any of the previous claims, wherein the mechanical stimuli sensor (2) is arranged on a distal phalanx zone (24) in relation to the palmar side of said glove (1).

5. The mechanical stimuli detection glove (1), according to any of the previous claims, wherein the mechanical stimuli sensor (2) is covered by an external second glove layer (21).

6. The mechanical stimuli detection glove (1), according to any of the previous claims, wherein at least one of the respective zones for insertion of a finger (23), namely the thumb finger, the index finger, the middle finger, the ring finger, and the little finger of said glove (1) comprises at least one mechanical stimuli sensor (2) adjacently connected to said respective zones for insertion of a finger (23).

7. The mechanical stimuli detection glove (1), according to any of the previous claims, wherein a connector (10), for instance a sewing line or an adhesive, connects the mechanical stimuli sensor (2) to at least a glove layer selected from the group consisting of an internal first glove layer (8), an external first glove layer (9), an external second glove layer (21) or an internal second glove layer (32).

8. The mechanical stimuli detection glove (1), according to any of the previous claims, wherein at least a hydrophobic insulating material (22) is deposited in at least one of the group consisting of the first fabric substrate layer (3), the second fabric substrate layer (6), and the third fabric substrate layer (29) to delimit the first layer zone impregnated with a hydrogel (4) or the second layer zone impregnated with hydrogel (30).

9. The mechanical stimuli detection glove (1), according to any of the previous claims, wherein the palm zone of said glove (1) comprises at least one rectangular array of a plurality of mechanical stimuli sensors (27), which are arranged in rows and columns.

10. The mechanical stimuli detection glove (1), according to any of the previous claims, wherein the mechanical stimuli sensor (2) is configured to enable the detection of at least one mechanical stimulus selected from a group consisting of a pressure, a touch, a strain, and an elongation.

11. The mechanical stimuli detection glove (1), according to any of the previous claims, wherein at least one of the first layer zone impregnated with a hydrogel (4) or the second layer zone impregnated with hydrogel (30) includes a hydrogel selected from the group consisting of a cellulose derivative, a polynucleotide, a polypeptide, a polysaccharide, a natural rubber, a polyphenolic polymer, a polyacrylamide, a complex of polymers of large chain fatty acids or their mixtures or their composites.

12. The mechanical stimuli detection glove (1), according to the previous claim, wherein the cellulose derivative hydrogel is selected from the group consisting of cellulose acetate, cellulose acetate phthalate, cellulose acetate butyrate, cellulose acetate trimellitate, hydroxypropyl methylcellulose phthalate, methylcellulose, ethyl cellulose, hydroxyethyl cellulose, carboxymethyl cellulose, sodium carboxymethyl cellulose, hydroxypropyl cellulose, hydroxypropyl methylcellulose, their composites or their composites with natural polymers, polyvinyl alcohol, polyelectrolyte complexes, interpenetrating polymer network, cellulose-inorganic hybrid hydrogels, or their mixtures or their composites.

13. The mechanical stimuli detection glove (1), according to any of the previous claims, wherein at least one of the first layer zone impregnated with a hydrogel (4) or the second layer zone impregnated with hydrogel (30) includes at least a salt in the hydrogel matrix, wherein said salt includes a cation selected from a group consisting of a monovalent cation, a divalent cation, or a trivalent cation.

14. The mechanical stimuli detection glove (1), according to any of the previous claims, wherein any one of the first fabric substrate layer (3), the second fabric substrate layer (6), the internal first glove layer (8), the external first glove layer (9), the external second glove layer (21), the third fabric substrate (29) or the internal second glove layer (32) is selected from a group consisting of a fibrous insulator woven fabric or a fibrous insulator unwoven fabric, for example a cotton fabric.

15. The mechanical stimuli detection glove (1), according to any of the previous claims, wherein at least one of the group consisting of the first sensor electrode (5), the second sensor electrode (7), the third sensor electrode (28), the first conductive line (11), the second conductive line (12), or the third conductive line (31) is made of a material selected from the group consisting of carbon, silver, gold, platinum, copper, aluminum, alloys thereof, or metallic alloys, for example stainless steel; or a conductive polymer or copolymer, for example polyaniline or poly (3,4-ethylenedioxythiophene) polystyrene sulfonate.

16. The mechanical stimuli detection glove (1), according to any of the previous claims, wherein a first conductive cable (25) is connected to the first conductive lines (11), a second conductive cable (26) is connected to the second conductive lines (12), and a third conductive cable is connected to the third conductive lines (31).

17. A method of preparation of a mechanical stimuli detection glove (1), as defined in any of the previous claims, **characterized by** comprising the following steps regarding the preparation of a mechanical stimuli sensor (2):
a) Depositing and impregnating a hydrogel in a first fabric substrate layer (3) forming a first layer zone impregnated with a hydrogel (4);
b) Connecting a portion of a first sensor electrode (5) to an upper part of said first layer zone impregnated with a hydrogel (4);
c) Connecting a portion of a second sensor electrode (7) to a lower part of said first layer zone impregnated with a hydrogel (4) and to an upper portion of a second fabric substrate layer (6);
d) Connecting a first conductive line (11) to the first sensor electrode (5);
e) Connecting a second conductive line (12) to the second sensor electrode (7);
and said method comprising the further following step:
f) Connecting the mechanical stimuli sensor (2) to at least one finger zone of said glove (1);
wherein the steps b) and c) are executed in any order between them, and the steps d) and e) are executed in any order between them.

18. The method of preparation of a mechanical stimuli detection glove (1), according to claim 17, wherein, before the step f), further comprises the following steps:
i) Depositing and impregnating a hydrogel in a second fabric substrate layer (6) forming a second layer zone impregnated with a hydrogel (30);
ii) Connecting a portion of the second sensor electrode (7) to an upper part of said second layer zone impregnated with a hydrogel (30).

19. The method of preparation of a mechanical stimuli detection glove (1), according to claim 17, wherein, before the step f), further comprises the following steps:
i) Depositing and impregnating a hydrogel in a third fabric substrate layer (29) forming a second layer zone impregnated with a hydrogel (30);
ii) Connecting a portion of a third sensor electrode (31) to an upper part of said second layer zone impregnated with a hydrogel (30);
iii) Connecting a portion of the third sensor electrode (31) to a lower part of said second fabric substrate layer (6).

20. The method of preparation of a mechanical stimuli detection glove (1), according to any of claims 17 to 19, wherein further comprises a step of connecting the mechanical stimuli sensor (2) to at least one of the group consisting of an internal first glove layer (8), an external first glove layer (9) or an internal second glove layer (32) forming a zone for insertion of a finger (23), and said mechanical stimuli sensor (2) being arranged at an adjacent position in relation to said zone for insertion of a finger (23), preferably in an end portion of said zone for insertion of a finger (23).

21. The method of preparation of a mechanical stimuli detection glove (1), according to any of the claims 17 to 20, wherein further comprises a step of covering the mechanical stimuli sensor (2) by an external second glove layer (21).

22. The method of preparation of a mechanical stimuli detection glove (1), according to any of the claims 17 to 21, wherein further comprises a step of depositing a hydrophobic insulating material (22) in at least one of the group consisting of the first fabric substrate layer (3), the second fabric substrate layer (6) or the third fabric substrate layer (29) to delimit the first layer zone impregnated with a hydrogel (4) or the second layer zone impregnated with a hydrogel (30).

23. A mechanical stimuli detection system **characterized by** comprising the mechanical stimuli detection glove (1), as defined in any of the claims 1 to 16, and further comprising:
a hybrid connector (13), which is connected to a plurality of first conductive lines (11) and to a plurality of second conductive lines (12) of said glove (1); and
a computing device (15), which is connected to said hybrid connector (13) by a computer connection cable (14);
wherein said computing device (15) comprises a microcontroller (16), which is configured to process data retrieved by the mechanical stimuli sensor (2) and transmits processed data to a data transmission and reception unit (19).

24. The mechanical stimuli detection system, according to the previous claim, wherein a power supply (20) comprises at least one of the group consisting of a charging unit (18) or a battery.

25. A non-therapeutic use of a mechanical stimuli detection system, as defined in any of the claims 23 to 24, as a mechanical stimuli monitoring system based on at least a feedback signal, which is applied to at least one element selected from the group consisting of a finger or a palm hand, wherein said feedback signal is an electrostimulation impulse imposed adjacently to a mechanical stimuli sensor (2) of the mechanical stimuli detection glove (1), as defined in any of the claims 1 to 16.

26. The non-therapeutic use of a mechanical stimuli detection system, according to the previous claim, wherein the electrostimulation impulse is an electrical current having its input through one component of a conductive group consisting of a second sensor electrode (7) or a third sensor electrode (28) and having its output through another component of said conductive group.

27. The non-therapeutic use of a mechanical stimuli detection system, according to the previous claim, wherein said use is related to at least one of the group consisting of a hand and/or fingers pressure monitoring, a haptic response monitoring, an evaluation of an improvement regarding performance and/or injuries prevention in sports, a connection with computational devices that provide experiences of virtual/augmented reality, or a connection with computational devices that provide human-machine interfaces.

## Patentansprüche

1. Mechanischer Stimulus Detektionshandschuh (1), der mindestens einen Sensor (2) für mechanische Reize umfasst, der mit mindestens einem Fingerbereich des Handschuhs (1) verbunden ist, wobei der Sensor (2) für mechanische Reize Folgendes umfasst:
eine erste Gewebesubstratschicht (3), die eine mit einem Hydrogel (4) imprägnierte erste Schicht umfasst; und
eine zweite Gewebesubstratschicht (6); und
eine erste Sensorelektrode (5), deren Abschnitt mit einem oberen Teil des mit einem Hydrogel (4) imprägnierten ersten Schichtbereichs verbunden ist; und
eine zweite Sensorelektrode (7), deren oberer Abschnitt mit einem unteren Teil des mit einem Hydrogel (4) imprägnierten ersten Schichtbereichs verbunden ist und deren unterer Abschnitt mit einem oberen Teil der zweiten Gewebesubstratschicht (6) verbunden ist; wobei
die erste Sensorelektrode (5) mit einer ersten leitfähigen Leitung (11) verbunden ist; und
die zweite Sensorelektrode (7) mit einer zweiten leitfähigen Leitung (12) verbunden ist; **dadurch gekennzeichnet, dass** die zweite Gewebesubstratschicht (6) einen zweiten, mit Hydrogel (30) imprägnierten Schichtbereich umfasst.

2. Mechanische Stimulus Detektionshandschuh (1) nach Anspruch 1, der ferner umfasst:
eine dritte Gewebesubstratschicht (29), die einen mit einem Hydrogel (30) imprägnierten zweiten Schichtbereich umfasst; und
eine dritte Sensorelektrode (28), deren oberer Abschnitt mit einem unteren Teil der zweiten Gewebesubstratschicht (6) verbunden ist und deren unterer Abschnitt mit einem oberen Abschnitt der mit einem Hydrogel (30) imprägnierten zweiten Schichtbereichs verbunden ist; wobei
die dritte Sensorelektrode (28) mit einer dritten leitfähigen Leitung (31) verbunden ist.

3. Mechanische Stimulus Detektionshandschuh (1) nach einem der vorstehenden Ansprüche, wobei der Sensor (2) für mechanische Reize mit mindestens einem Element aus der Gruppe verbunden ist, die aus einer inneren ersten Handschuhschicht (8), einer äußeren ersten Handschicht (9) oder einer inneren zweiten Handschicht (32) verbunden ist, die einen Bereich zum Einführen eines Fingers (23) bilden, wobei der Sensor (2) zur Erfassung mechanischer Reize an einer an diesen Bereich zum Einführen eines Fingers (23) angrenzenden Stelle, vorzugsweise in einem Endabschnitt dieses Bereichs zum Einführen eines Fingers (23), angeordnet ist.

4. Mechanische Stimulus Detektionshandschuh (1) nach einem der vorstehenden Ansprüche, wobei der Sensor für mechanische Reize (2) in Bezug auf die Handinnenseite des Handschuhs (1) an einem Bereich der distalen Phalanx (24) angeordnet ist.

5. Mechanische Stimulus Detektionshandschuh (1) nach einem der vorstehenden Ansprüche, wobei der Sensor (2) für mechanische Reize von einer äußeren zweiten Handschuh-Schicht (21) bedeckt ist.

6. Mechanische Stimulus Detektionshandschuh (1) nach einem der vorstehenden Ansprüche, wobei mindestens eine der jeweiligen Fingeröffnungen (23), nämlich die für Daumen, Zeigefinger, Mittelfinger, Ringfinger und kleinen Finger des Handschuhs (1), mindestens einen Sensor (2) zur Erfassung mechanischer Reize umfasst, der unmittelbar an die jeweilige Fingeröffnung angeschlossen ist (23) angrenzend verbunden ist.

7. Mechanische Stimulus Detektionshandschuh (1), nach einem der vorstehenden Ansprüche, wobei ein Verbindungselement (10), beispielsweise eine Naht oder ein Klebstoff, den Sensor (2) für mechanische Reize mit mindestens einer Handschuh-Schicht verbindet, die aus der Gruppe ausgewählt ist, bestehend aus einer inneren ersten Handschuh-Schicht (8), einer äußeren ersten Handschuh-Schicht (9), einer äußeren zweiten Handschuh-Schicht (21) oder einer inneren zweiten Handschuh-Schicht (32).

8. Mechanische Stimulus Detektionshandschuh (1), nach einem der vorstehenden Ansprüche, wobei mindestens ein hydrophobes Isoliermaterial (22) in mindestens einer der folgenden Schichten aufgebracht ist: der ersten Gewebesubstratschicht (3), der zweiten Gewebesubstratschicht (6) und der dritten Gewebesubstratschicht (29), um den mit einem Hydrogel (4) imprägnierten ersten Schichtbereich oder den mit Hydrogel (30) imprägnierten zweiten Schichtbereich abzugrenzen**.**

9. Mechanische Stimulus Detektionshandschuh (1) nach einem der vorstehenden Ansprüche, wobei die Handflächenzone des Handschuhs (1) mindestens eine rechteckige Anordnung einer Vielzahl mechanischer Reizsensoren (27) umfasst, die in Reihen und Spalten angeordnet sind.

10. Mechanischer Stimulus Detektionshandschuh (1) nach einem der vorstehenden Ansprüche, wobei der Sensor (2) für mechanische Reize so konfiguriert ist, dass er die Erkennung mindestens eines mechanischen Reizes ermöglicht, der aus einer Gruppe ausgewählt ist, die aus einem Druck, einer Berührung, einer Dehnung und einer Dehnung besteht.

11. Mechanische Stimulus Detektionshandschuh (1) nach einem der vorstehenden Ansprüche, wobei mindestens einer der mit einem Hydrogel (4) imprägnierten Bereiche der ersten Schicht oder der mit Hydrogel (30) imprägnierten Bereiche der zweiten Schicht ein Hydrogel enthält, das aus der Gruppe ausgewählt ist, bestehend aus einem Cellulosederivat, einem Polynukleotid, einem Polypeptid, einem Polysaccharid, einem Naturkautschuk, einem polyphenolischen Polymer, einem Polyacrylamid, einem Komplex aus Polymeren langkettiger Fettsäuren oder deren Mischungen oder deren Verbundstoffen besteht.

12. Mechanische Stimulus Detektionshandschuh (1) nach dem vorstehenden Anspruch, wobei das Cellulosederivat-Hydrogel aus der Gruppe ausgewählt ist, die aus Celluloseacetat, Celluloseacetatphthalat, Celluloseacetatbutyrat, Celluloseacetattrimellitat, Hydroxypropylmethylcellulosephthalat, Methylcellulose, Ethylcellulose, Hydroxyethylcellulose, Carboxymethylcellulose, Natriumcarboxymethylcellulose, Hydroxypropylcellulose, Hydroxypropylmethylcellulose, deren Verbundstoffe oder deren Verbundstoffe mit natürlichen Polymeren, Polyvinylalkohol, Polyelektrolytkomplexen, interpenetrierenden Polymernetzwerken, Zellulose-Anorganik-Hybridhydrogelen oder deren Mischungen oder deren Verbundstoffen ausgewählt ist.

13. Mechanische Stimulus Detektionshandschuh (1) nach einem der vorstehenden Ansprüche, wobei mindestens einer der mit einem Hydrogel (4) imprägnierten Bereiche der ersten Schicht oder der mit einem Hydrogel (30) imprägnierten Bereiche der zweiten Schicht mindestens ein Salz in der Hydrogelmatrix enthält, wobei das Salz ein Kation umfasst, das aus einer Gruppe ausgewählt ist, die aus einem einwertigen Kation, einem zweiwertigen Kation oder einem dreiwertigen Kation besteht.

14. Mechanische Stimulus Detektionshandschuh (1) gemäß einem der vorstehenden Ansprüche, wobei eine der folgenden Schichten: die erste Gewebesubstratschicht (3), die zweite Gewebesubstratschicht (6), die innere erste Handschuhschicht (8), die äußere erste Handschuhschicht (9), die äußere zweite Handschuhschicht (21), das dritte Gewebesubstrat (29) oder die innere zweite Handschuhschicht (32) aus einer Gruppe ausgewählt ist, die aus einem gewebten Faserisolatorgewebe oder einem nicht gewebten Faserisolatorgewebe, beispielsweise einem Baumwollgewebe, besteht..

15. Mechanische Stimulus Detektionshandschuh (1) gemäß einem der vorstehenden Ansprüche, wobei mindestens eines der Elemente aus der Gruppe bestehend aus der ersten Sensorelektrode (5), der zweiten Sensorelektrode (7), der dritten Sensorelektrode (28), der ersten leitfähigen Leitung (11), der zweiten leitfähigen Leitung (12) oder der dritten leitfähigen Leitung (31) aus einem Material besteht, das aus der Gruppe ausgewählt ist, die aus Kohlenstoff, Silber, Gold, Platin, Kupfer, Aluminium, deren Legierungen oder metallischen Legierungen, beispielsweise Edelstahl, besteht; oder aus einem leitfähigen Polymer oder Copolymer, beispielsweise Polyanilin oder Poly(3,4-ethylendioxythiophen)-polystyrolsulfonat.

16. Mechanische Stimulus Detektionshandschuh (1) gemäß einem der vorstehenden Ansprüche, wobei ein erstes leitfähiges Kabel (25) mit den ersten leitfähigen Leitungen (11) verbunden ist, ein zweites leitfähiges Kabel (26) mit den zweiten leitfähigen Leitungen (12) verbunden ist und ein drittes leitfähiges Kabel mit den dritten leitfähigen Leitungen (31) verbunden ist.

17. Verfahren zur Herstellung eines Mechanischen Stimulus Detektionshandschuhs (1), wie in einem der vorstehenden Ansprüche definiert, **gekennzeichnet durch** die folgenden Schritte zur Herstellung eines Sensors (2) zur Erfassung mechanischer Reize:
a) Aufbringen und Imprägnieren eines Hydrogels in einer ersten Gewebesubstratschicht (3), wodurch ein mit einem Hydrogel (4) imprägnierter erster Schichtbereich gebildet wird;
b) Verbinden eines Abschnitts einer ersten Sensorelektrode (5) mit einem oberen Teil des mit einem Hydrogel (4) imprägnierten ersten Schichtbereichs;
c) Verbinden eines Abschnitts einer zweiten Sensorelektrode (7) mit einem unteren Teil der mit einem Hydrogel (4) imprägnierten ersten Schicht (4) sowie mit einem oberen Abschnitt einer zweiten Gewebesubstratschicht (6);
d) Verbinden einer ersten leitfähigen Leitung (11) mit der ersten Sensorelektrode (5);
e) Verbinden einer zweiten leitfähigen Leitung (12) mit der zweiten Sensorelektrode (7);
und das Verfahren den folgenden weiteren Schritt umfasst:
f) Verbinden des Sensors (2) für mechanische Reize mit mindestens einem Fingerbereich des Handschuhs (1);
wobei die Schritte b) und c) in beliebiger Reihenfolge zueinander ausgeführt werden und die Schritte d) und e) in beliebiger Reihenfolge zueinander ausgeführt werden.

18. Verfahren zur Herstellung eines Mechanischen Stimulus Detektionshandschuhs (1) gemäß Anspruch 17, wobei vor dem Schritt f) zusätzlich die folgenden Schritte umfasst:
i) Aufbringen und Imprägnieren eines Hydrogels in einer zweiten Gewebesubstratschicht (6), wodurch ein mit einem Hydrogel (30) imprägnierter zweiter Schichtbereich gebildet wird;
ii) Verbinden eines Abschnitts der zweiten Sensorelektrode (7) mit einem oberen Teil des genannten, mit einem Hydrogel (30) imprägnierten zweiten Schichtbereichs.

19. Verfahren zur Herstellung eines Mechanischen Stimulus Detektionshandschuhs (1) gemäß Anspruch 17, wobei vor dem Schritt f) zusätzlich die folgenden Schritte umfasst:
i) Verbinden eines Abschnitts einer dritten Sensorelektrode (31) mit einem oberen Teil der mit einem Hydrogel (30) imprägnierten zweiten Schichtbereich;
ii) Verbinden eines Abschnitts einer dritten Sensorelektrode (31) mit einem oberen Teil der mit einem Hydrogel (30) imprägnierten zweiten Schichtbereich;
iii) Verbinden eines Abschnitts der dritten Sensorelektrode (31) mit einem unteren Teil der zweiten Gewebesubstratschicht (6).

20. Verfahren zur Herstellung eines Mechanischen Stimulus Detektionshandschuhs (1) gemäß einem der Ansprüche 17 bis 19, wobei das Verfahren ferner einen Schritt umfasst, bei dem der Sensor (2) für mechanische Reize mit mindestens einem Element aus der Gruppe verbunden wird, die aus einer inneren ersten Handschuhschicht (8), einer äußeren ersten Handschuhschicht (9) oder einer inneren zweiten Handschuhschicht (32) besteht, die einen Bereich zum Einführen eines Fingers (23) bildet, und wobei der Sensor für mechanische Reize (2) an einer an diesen Bereich zum Einführen eines Fingers (23) angrenzenden Position angeordnet ist, vorzugsweise in einem Endabschnitt dieses Bereichs zum Einführen eines Fingers (23).

21. Verfahren zur Herstellung eines Mechanischen Stimulus Detektionshandschuhs (1) gemäß einem der Ansprüche 17 bis 20, wobei das Verfahren ferner einen Schritt umfasst, bei dem der Sensor für mechanische Reize (2) durch eine äußere zweite Handschicht (21) abgedeckt wird.

22. Verfahren zur Herstellung eines Mechanischen Stimulus Detektionshandschuhs (1) gemäß einem der Ansprüche 17 bis 21, wobei das Verfahren ferner einen Schritt umfasst, bei dem ein hydrophobes Isoliermaterial (22) in mindestens einer der folgenden Schichten aufgebracht wird: der ersten Gewebesubstratschicht (3), der zweiten Gewebesubstratschicht (6) oder der dritten Gewebesubstratschicht (29) umfasst, um den mit einem Hydrogel (4) imprägnierten ersten Schichtbereich oder den mit einem Hydrogel (30) imprägnierten zweiten Schichtbereich abzugrenzen.

23. Ein System zur Erfassung mechanischer Reize, **gekennzeichnet dadurch, dass** es den Mechanischen Stimulus Detektionshandschuh (1) zur Erfassung mechanischer Reize, wie in einem der Ansprüche 1 bis 16 definiert, umfasst und ferner Folgendes umfasst:
einen Hybridstecker (13), der mit einer Vielzahl von ersten Leiterbahnen (11) und einer Vielzahl von zweiten Leiterbahnen (12) des Handschuhs (1) verbunden ist; und
eine Vorrichtung (15), die über ein Computer-Verbindungskabel (14) mit dem Hybridstecker (13) verbunden ist;
wobei die Vorrichtung (15) einen Mikrocontroller (16) umfasst, der dazu ausgelegt ist, vom Sensor (2) für mechanische Reize erfasste Daten zu verarbeiten und die verarbeiteten Daten an eine Datenübertragungs- und - empfangseinheit (19) zu übertragen.

24. Das System zur Erfassung mechanischer Reize gemäß dem vorstehenden Anspruch, wobei eine Stromversorgung (20) mindestens ein Element aus der Gruppe bestehend aus einer Ladeeinheit (18) oder einer Batterie umfasst.

25. Eine nichttherapeutische Verwendung eines Systems zur Erfassung mechanischer Reize, wie in einem der Ansprüche 23 bis 24 definiert, als Überwachungssystem für mechanische Reize auf der Grundlage mindestens eines Rückkopplungssignals, das an mindestens ein Element angelegt wird, das aus der Gruppe ausgewählt ist, die aus einem Finger oder einer Handfläche besteht, wobei das Rückkopplungssignal ein Elektrostimulationsimpuls ist, der in unmittelbarer Nähe eines Sensors (2) für mechanische Reize des Mechanischen Stimulus Detektionshandschuhs (1), wie in einem der Ansprüche 1 bis 16 definiert, aufgebracht wird.

26. Die nichttherapeutische Verwendung eines Systems zur Erfassung mechanischer Reize gemäß dem vorstehenden Anspruch, wobei der Elektrostimulationsimpuls ein elektrischer Strom ist, der über eine Komponente einer leitfähigen Gruppe, bestehend aus einer zweiten Sensorelektrode (7) oder einer dritten Sensorelektrode (28), eingespeist wird und über eine andere Komponente der genannten leitfähigen Gruppe wieder abgegeben wird.

27. Die nichttherapeutische Verwendung eines Systems zur Erfassung mechanischer Reize gemäß dem vorstehenden Anspruch, wobei sich diese Verwendung auf mindestens einen der folgenden Punkte bezieht: die Überwachung des Drucks an der Hand und/oder den Fingern, die Überwachung haptischer Reaktionen, die Bewertung von Leistungsverbesserungen und/oder die Verletzungsprävention im Sport, die Verbindung mit Vorrichtungen, die Erfahrungen im Bereich der virtuellen/erweiterten Realität ermöglichen, oder die Verbindung mit Vorrichtungen, die Mensch-Maschine-Schnittstellen bereitstellen..

## Revendications

1. Un gant de détection de stimuli mécaniques (1) comprenant au moins un capteur de stimuli mécaniques (2) connecté à au moins une zone de doigt dudit gant (1), **caractérisé en ce que** ledit capteur de stimuli mécaniques (2) comprend :
une première couche de substrat textile (3), qui comprend une première zone de couche imprégnée d'un hydrogel (4) ; et
une deuxième couche de substrat textile (6) ; et
une première électrode de capteur (5) ayant une portion connectée à une partie supérieure de ladite première zone de couche imprégnée d'un hydrogel (4) ; et
une deuxième électrode de capteur (7) ayant une portion supérieure connectée à une partie inférieure de ladite première zone de couche imprégnée d'un hydrogel (4) et une portion inférieure connectée à une portion supérieure de ladite deuxième couche de substrat textile (6) ; dans lequel
la première électrode de capteur (5) est connectée à une première ligne conductrice (11) ; et
la deuxième électrode de capteur (7) est connectée à une deuxième ligne conductrice (12) ; **caractérisé en ce que** la deuxième couche de substrat textile (6) comprend une deuxième zone de couche imprégnée d'un hydrogel (30).

2. Le gant de détection de stimuli mécaniques (1), selon la revendication 1, **caractérisé en ce qu'**il comprend en outre :
une troisième couche de substrat textile (29), qui comprend une deuxième zone de couche imprégnée d'un hydrogel (30) ; et
une troisième électrode de capteur (28) ayant une portion supérieure connectée à une partie inférieure de la deuxième couche de substrat textile (6) et une portion inférieure connectée à une portion supérieure de ladite deuxième zone de couche imprégnée d'un hydrogel (30) ; dans lequel
la troisième électrode de capteur (28) est connectée à une troisième ligne conductrice (31).

3. Le gant de détection de stimuli mécaniques (1), selon l'une quelconque des revendications précédentes, **caractérisé en ce que** le capteur de stimuli mécaniques (2) est connecté à au moins un élément du groupe constitué d'une première couche interne de gant (8), d'une première couche externe de gant (9) ou d'une deuxième couche interne de gant (32) formant une zone d'insertion d'un doigt (23), dans lequel ledit capteur de stimuli mécaniques (2) est disposé à une position adjacente par rapport à ladite zone d'insertion d'un doigt (23), de préférence dans une portion d'extrémité de ladite zone d'insertion d'un doigt (23).

4. Le gant de détection de stimuli mécaniques (1), selon l'une quelconque des revendications précédentes, **caractérisé en ce que** le capteur de stimuli mécaniques (2) est disposé sur une zone de phalange distale (24) par rapport au côté palmaire dudit gant (1).

5. Le gant de détection de stimuli mécaniques (1), selon l'une quelconque des revendications précédentes, **caractérisé en ce que** le capteur de stimuli mécaniques (2) est recouvert d'une deuxième couche externe de gant (21).

6. Le gant de détection de stimuli mécaniques (1), selon l'une quelconque des revendications précédentes, **caractérisé en ce qu'**au moins une des zones respectives d'insertion d'un doigt (23), à savoir le pouce, l'index, le majeur, l'annulaire et l'auriculaire dudit gant (1), comprend au moins un capteur de stimuli mécaniques (2) connecté de manière adjacente auxdites zones respectives d'insertion d'un doigt (23).

7. Le gant de détection de stimuli mécaniques (1), selon l'une quelconque des revendications précédentes, **caractérisé en ce qu'**un connecteur (10), par exemple une ligne de couture ou un adhésif, connecte le capteur de stimuli mécaniques (2) à au moins une couche de gant sélectionnée dans le groupe constitué d'une première couche interne de gant (8), d'une première couche externe de gant (9), d'une deuxième couche externe de gant (21) ou d'une deuxième couche interne de gant (32).

8. Le gant de détection de stimuli mécaniques (1), selon l'une quelconque des revendications précédentes, **caractérisé en ce qu'**au moins un matériau isolant hydrophobe (22) est déposé dans au moins un élément du groupe constitué de la première couche de substrat textile (3), de la deuxième couche de substrat textile (6) et de la troisième couche de substrat textile (29) pour délimiter la première zone de couche imprégnée d'un hydrogel (4) ou la deuxième zone de couche imprégnée d'un hydrogel (30).

9. Le gant de détection de stimuli mécaniques (1), selon l'une quelconque des revendications précédentes, **caractérisé en ce que** la zone de paume dudit gant (1) comprend au moins un réseau rectangulaire d'une pluralité de capteurs de stimuli mécanique (27), qui sont disposés en rangées et en colonnes.

10. Le gant de détection de stimuli mécaniques (1), selon l'une quelconque des revendications précédentes, **caractérisé en ce que** le capteur de stimuli mécaniques (2) est configuré pour permettre la détection d'au moins un stimulus mécanique sélectionné dans un groupe constitué d'une pression, d'un contact tactile, d'une déformation et d'un allongement.

11. Le gant de détection de stimuli mécaniques (1), selon l'une quelconque des revendications précédentes, **caractérisé en ce qu'**au moins l'une de la première zone de couche imprégnée d'un hydrogel (4) ou de la deuxième zone de couche imprégnée d'un hydrogel (30) inclut un hydrogel sélectionné dans le groupe constitué d'un dérivé de cellulose, d'un polynucléotide, d'un polypeptide, d'un polysaccharide, d'un caoutchouc naturel, d'un polymère polyphénolique, d'un polyacrylamide, d'un complexe de polymères d'acides gras à longue chaîne ou de leurs mélanges ou de leurs composites.

12. Le gant de détection de stimuli mécaniques (1), selon la revendication précédente, **caractérisé en ce que** l'hydrogel dérivé de cellulose est sélectionné dans le groupe constitué d'acétate de cellulose, d'acétate phtalate de cellulose, d'acétate butyrate de cellulose, d'acétate trimellitate de cellulose, de phtalate d'hydroxypropylméthylcellulose, de méthylcellulose, d'éthylcellulose, d'hydroxyéthylcellulose, de carboxyméthylcellulose, de carboxyméthylcellulose sodique, d'hydroxypropylcellulose, d'hydroxypropylméthylcellulose, de leurs composites ou de leurs composites avec des polymères naturels, d'alcool polyvinylique, de complexes polyélectrolytiques, d'un réseau de polymères interpénétrés, d'hydrogels hybrides cellulose-inorganique, ou de leurs mélanges ou de leurs composites.

13. Le gant de détection de stimuli mécaniques (1), selon l'une quelconque des revendications précédentes, **caractérisé en ce qu'**au moins l'une de la première zone de couche imprégnée d'un hydrogel (4) ou de la deuxième zone de couche imprégnée d'un hydrogel (30) inclut au moins un sel dans la matrice d'hydrogel, dans lequel ledit sel inclut un cation sélectionné dans un groupe constitué d'un cation monovalent, d'un cation divalent ou d'un cation trivalent.

14. Le gant de détection de stimuli mécaniques (1), selon l'une quelconque des revendications précédentes, **caractérisé en ce que** l'un quelconque des éléments suivants : la première couche de substrat textile (3), la deuxième couche de substrat textile (6), la première couche interne de gant (8), la première couche externe de gant (9), la deuxième couche externe de gant (21), le troisième substrat textile (29) ou la deuxième couche interne de gant (32), est sélectionné dans un groupe constitué d'un tissu tissé isolant fibreux ou d'un tissu non tissé isolant fibreux, par exemple un tissu de coton.

15. Le gant de détection de stimuli mécaniques (1), selon l'une quelconque des revendications précédentes, **caractérisé en ce qu'**au moins un élément du groupe constitué de la première électrode de capteur (5), de la deuxième électrode de capteur (7), de la troisième électrode de capteur (28), de la première ligne conductrice (11), de la deuxième ligne conductrice (12) ou de la troisième ligne conductrice (31) est réalisé en un matériau sélectionné dans le groupe constitué de carbone, d'argent, d'or, de platine, de cuivre, d'aluminium, de leurs alliages ou d'alliages métalliques, par exemple de l'acier inoxydable ; ou d'un polymère ou copolymère conducteur, par exemple de la polyaniline ou du poly(3,4-éthylènedioxythiophène) polystyrène sulfonate.

16. Le gant de détection de stimuli mécaniques (1), selon l'une quelconque des revendications précédentes, **caractérisé en ce qu'**un premier câble conducteur (25) est connecté aux premières lignes conductrices (11), un deuxième câble conducteur (26) est connecté aux deuxièmes lignes conductrices (12), et un troisième câble conducteur est connecté aux troisièmes lignes conductrices (31).

17. Un procédé de préparation d'un gant de détection de stimuli mécaniques (1), tel que défini dans l'une quelconque des revendications précédentes, **caractérisé en ce qu'**il comprend les étapes suivantes concernant la préparation d'un capteur de stimuli mécaniques (2) :
a) Déposer et imprégner un hydrogel dans une première couche de substrat textile (3) formant une première zone de couche imprégnée d'un hydrogel (4) ;
b) Connecter une portion d'une première électrode de capteur (5) à une partie supérieure de ladite première zone de couche imprégnée d'un hydrogel (4) ;
c) Connecter une portion d'une deuxième électrode de capteur (7) à une partie inférieure de ladite première zone de couche imprégnée d'un hydrogel (4) et à une portion supérieure d'une deuxième couche de substrat textile (6) ;
d) Connecter une première ligne conductrice (11) à la première électrode de capteur (5) ;
e) Connecter une deuxième ligne conductrice (12) à la deuxième électrode de capteur (7) ;
et ledit procédé comprenant en outre l'étape suivante :
f) Connecter le capteur de stimuli mécaniques (2) à au moins une zone de doigt dudit gant (1) ;
dans lequel les étapes b) et c) sont exécutées dans un ordre quelconque entre elles, et les étapes d) et ) sont exécutées dans un ordre quelconque entre elles.

18. Le procédé de préparation d'un gant de détection de stimuli mécaniques (1), selon la revendication 17, **caractérisé en ce qu'**il comprend en outre, avant l'étape f), les étapes suivantes :
i) Déposer et imprégner un hydrogel dans une deuxième couche de substrat textile (6) formant une deuxième zone de couche imprégnée d'un hydrogel (30) ;
ii) Connecter une portion de la deuxième électrode de capteur (7) à une partie supérieure de ladite deuxième zone de couche imprégnée d'un hydrogel (30).

19. Le procédé de préparation d'un gant de détection de stimuli mécaniques (1), selon la revendication 17, **caractérisé en ce qu'**il comprend en outre, avant l'étape f), les étapes suivantes :
i) Déposer et imprégner un hydrogel dans une troisième couche de substrat textile (29) formant une deuxième zone de couche imprégnée d'un hydrogel (30) ;
ii) Connecter une portion d'une troisième électrode de capteur (31) à une partie supérieure de ladite deuxième zone de couche imprégnée d'un hydrogel (30) ;
iii) Connecter une portion de la troisième électrode de capteur (31) à une partie inférieure de ladite deuxième couche de substrat textile (6).

20. Le procédé de préparation d'un gant de détection de stimuli mécaniques (1), selon l'une quelconque des revendications 17 à 19, **caractérisé en ce qu'**il comprend en outre une étape de connexion du capteur de stimuli mécaniques (2) à au moins un élément du groupe constitué d'une première couche interne de gant (8), d'une première couche externe de gant (9) ou d'une deuxième couche interne de gant (32) formant une zone d'insertion d'un doigt (23), et ledit capteur de stimuli mécaniques (2) étant disposé à une position adjacente par rapport à ladite zone d'insertion d'un doigt (23), de préférence dans une portion d'extrémité de ladite zone d'insertion d'un doigt (23).

21. Le procédé de préparation d'un gant de détection de stimuli mécaniques (1), selon l'une quelconque des revendications 17 à 20, **caractérisé en ce qu'**il comprend en outre une étape de recouvrement du capteur de stimuli mécaniques (2) par une deuxième couche externe de gant (21).

22. Le procédé de préparation d'un gant de détection de stimuli mécaniques (1), selon l'une quelconque des revendications 17 à 21, **caractérisé en ce qu'**il comprend en outre une étape de dépôt d'un matériau isolant hydrophobe (22) dans au moins un élément du groupe constitué de la première couche de substrat textile (3), de la deuxième couche de substrat textile (6) ou de la troisième couche de substrat textile (29) pour délimiter la première zone de couche imprégnée d'un hydrogel (4) ou la deuxième zone de couche imprégnée d'un hydrogel (30).

23. Un système de détection de stimuli mécaniques **caractérisé en ce qu'**il comprend le gant de détection de stimuli mécaniques (1), tel que défini dans l'une quelconque des revendications 1 à 16, et comprend en outre :
un connecteur hybride (13), qui est connecté à une pluralité de premières lignes conductrices (11) et à une pluralité de deuxièmes lignes conductrices (12) dudit gant (1) ; et
un dispositif informatique (15), qui est connecté audit connecteur hybride (13) par un câble de connexion informatique (14) ;
dans lequel ledit dispositif informatique (15) comprend un microcontrôleur (16), qui est configuré pour traiter les données recueillies par le capteur de stimuli mécaniques (2) et transmet les données traitées à une unité de transmission et de réception de données (19).

24. Le système de détection de stimuli mécaniques, selon la revendication précédente, **caractérisé en ce qu'**une alimentation électrique (20) comprend au moins un élément du groupe constitué d'une unité de charge (18) ou d'une batterie.

25. Une utilisation non thérapeutique d'un système de détection de stimuli mécaniques, tel que défini dans l'une quelconque des revendications 23 à 24, en tant que système de surveillance de stimuli mécaniques basé sur au moins un signal de rétroaction, qui est appliqué à au moins un élément sélectionné dans le groupe constitué d'un doigt ou d'une paume de main, **caractérisée en ce que** ledit signal de rétroaction est une impulsion d'électrostimulation imposée de manière adjacente à un capteur de stimuli mécaniques (2) du gant de détection de stimuli mécaniques (1), tel que défini dans l'une quelconque des revendications 1 à 16.

26. Une utilisation non thérapeutique d'un système de détection de stimuli mécaniques, selon la revendication précédente, **caractérisée en ce que** l'impulsion d'électrostimulation est un courant électrique ayant son entrée par l'un des composants d'un groupe conducteur constitué d'une deuxième électrode de capteur (7) ou d'une troisième électrode de capteur (28) et ayant sa sortie par un autre composant dudit groupe conducteur.

27. Une utilisation non thérapeutique d'un système de détection de stimuli mécaniques, selon la revendication précédente, **caractérisée en ce que** ladite utilisation est liée à au moins un élément du groupe constitué d'une surveillance de la pression de la main et/ou des doigts, d'une surveillance de la réponse haptique, d'une évaluation d'une amélioration concernant les performances et/ou la prévention des blessures dans le sport, d'une connexion avec des dispositifs de calcul qui fournissent des expériences de réalité virtuelle/augmentée, ou d'une connexion avec des dispositifs de calcul qui fournissent des interfaces homme-machine.
